# EUROPEAN PATENT SPECIFICATION

(11) **EP 4 696 220 B1**
(45) Date of publication and mention of the grant of the patent: **30.09.2026**
(21) Application number: 24194537.7
(22) Date of filing: 14.08.2024
(51) Int. Cl.: A61B 3/10

(54) **PATIENT ALIGNMENT SYSTEM FOR AN OPHTHALMIC IMAGING INSTRUMENT**
PATIENTENAUSRICHTUNGSSYSTEM FÜR EIN OPHTHALMISCHES BILDGEBUNGSINSTRUMENT
SYSTÈME D'ALIGNEMENT DE PATIENT POUR UN INSTRUMENT D'IMAGERIE OPHTALMIQUE

(43) Date of publication of application: 18.02.2026
(73) Proprietor: Optos plc, Dunfermline, Scotland KY11 8GR (GB)
(72) Inventor: PINNNOCK, Ralph, Dunfermline, Scotland, KY11 8GR (GB); LEE, Andrew, Dunfermline, Scotland, KY11 8GR (GB); PEMBERTON, Stephen, Dunfermline, Scotland, KY11 8GR (GB)
(74) Representative: Hoffmann Eitle

(56) References cited:
- US-A1- 2016 089 024
- US-A1- 2017 347 882
- US-A1- 2020 237 217

## Description

### [Field]

The present invention generally relate to the field of ophthalmic imaging scanners and, more particularly, to systems for bringing an eye of a subject into a position for acquiring an image of a fundus of the eye by an ophthalmic imaging scanner.

### [Background]

Ophthalmic imaging scanners employ various techniques to image different parts of an eye of a subject, and are used by clinicians to diagnose and manage a variety of eye conditions. Ophthalmic imaging scanners acquire images of the fundus or other part of the eye by scanning a beam of light across the eye and detecting return light from the eye. To acquire high quality images of the fundus of the eye, ophthalmic imaging scanners, such as scanning laser ophthalmoscopes (SLOs) and optical coherence tomography (OCT) scanners, for example, often require the pupil of the eye to fall within a predetermined range of distances from an exit pupil (or other reference position) of the ophthalmic imaging scanner that are suitable for acquiring such images. Pupil alignment can be particularly important for widefield (WF) and ultra-widefield (UWF) ophthalmic imaging scanners, where the acquisition of a WF or UWF fundus image relies on light propagating to and from the fundus, through the pupil, at widely varying angles of incidence on the pupillary plane.

For example, US 2020/237217 A1 discloses an ophthalmic device including an illumination module which scans light across a region of the retina of an eye when the pupil is disposed at a focal point of the illumination module. The ophthalmic device further comprises components for: aligning the pupil with the focal point; monitoring a position of the pupil relative to the focal point and maintaining the alignment based on the monitored position; aligning a scan location of the illumination module on the retina to a target scan location while the alignment of the pupil with the focal point is being maintained, wherein the illumination module performs scan at the target scan location The ophthalmic device further maintains the scan location at the target scan location while the alignment of the pupil with the focal point is being maintained, using scan location correction information based on retinal feature information.

Stereoscopic ranging techniques employing stereo cameras are often used to measure distance to the eye. For example, some ophthalmic imaging scanners include a so-called pupil alignment module (PAM), which comprises stereo cameras to acquire stereo images of the eye, and is arranged to determine the distance between the PAM and the pupil based on a separation between the pupil centres in the stereo images. Visual feedback based on the determined distance is provided to guide the subject in bringing their eye into a position suitable for imaging the eye.

### [Summary]

The present invention provides an ultra-widefield ophthalmic imaging instrument according to claim 1, an ultra-widefield ophthalmic imaging instrument according to claim 3, a method of operating an ultra-widefield ophthalmic imaging instrument according to claim 9, and a method of operating an ultra-widefield ophthalmic imaging instrument according to claim 11. Optional features are set out in the dependent claims.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an ultra-widefield ophthalmic imaging instrument according to an example embodiment herein.
Figure 2 is a schematic illustration of an example implementation of the ultra-widefield ophthalmic imaging instrument of the example embodiment of Figure 1.
Figure 3 is a schematic illustration of a programmable signal processing hardware, which may be configured to perform the functions of the processor 50 described herein.
Figure 4 is schematic showing a cross-section of the ellipsoidal mirror 32 of the example implementation in a plane which is perpendicular to the axis of symmetry of the ellipsoidal mirror and includes the first focal point 32-1, as well as top view of the stereo imaging apparatus 40 and fixation target light source 80 of the example implementation, along the axis of symmetry.
Figure 5 is a schematic illustration of an ultra-widefield ophthalmic imaging instrument according to a variant of the example implementation herein, which comprises two ellipsoidal mirrors having a shared focal point, wherein the rotating mirror 30 is provided at the remaining focal point of one of the ellipsoidal mirrors to scan line-field illumination across the fundus 12 via the pupil 14 provided at the remaining focal point of the other ellipsoidal mirror.
Figure 6 is a schematic illustration of a pupil alignment device of an UWF ophthalmic imaging instrument of an example embodiment, when viewed from the rotatable mirror 30. The pupil alignment device comprises the stereo imaging system 40 and a modified version of the fixation target light source 80 included in the example implementation of Figure 2.
Figure 7 is a flow diagram illustrating a method of operating an UWF ophthalmic imaging instrument to acquire an UWF fundus image in accordance with an example embodiment.
Figure 8 is a flow diagram illustrating an example of how the position of the pupil may be compared with the range of distances from the exit pupil suitable for acquiring the fundus image to determine whether the pupil is within the range.
Figure 9 is a schematic illustration of an alternative implementation of the patient alignment device of the example embodiment.
Figure 10 is a schematic illustration of a further alternative implementation of the patient alignment device of the example embodiment.

### [Detailed Description of Example Embodiments]

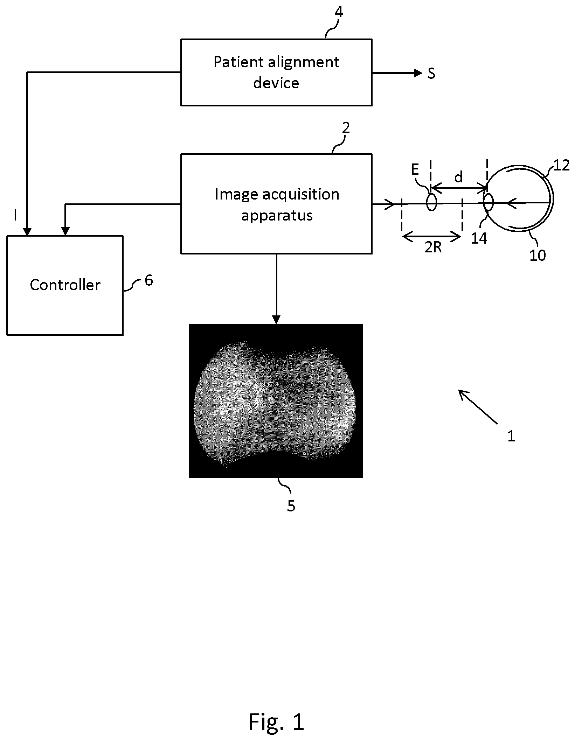

Figure 1 is a schematic illustration of an ophthalmic imaging instrument 1, which includes an image acquisition apparatus 2 arranged to acquire an image 5 of a fundus 12 of an eye 10 of a subject via an exit pupil E of the image acquisition apparatus 2. The ocular fundus 12 is the inner lining of the eye 10 opposite the lens, and comprises the retina, macula, optic disc, fovea, choroid, and blood vessels.

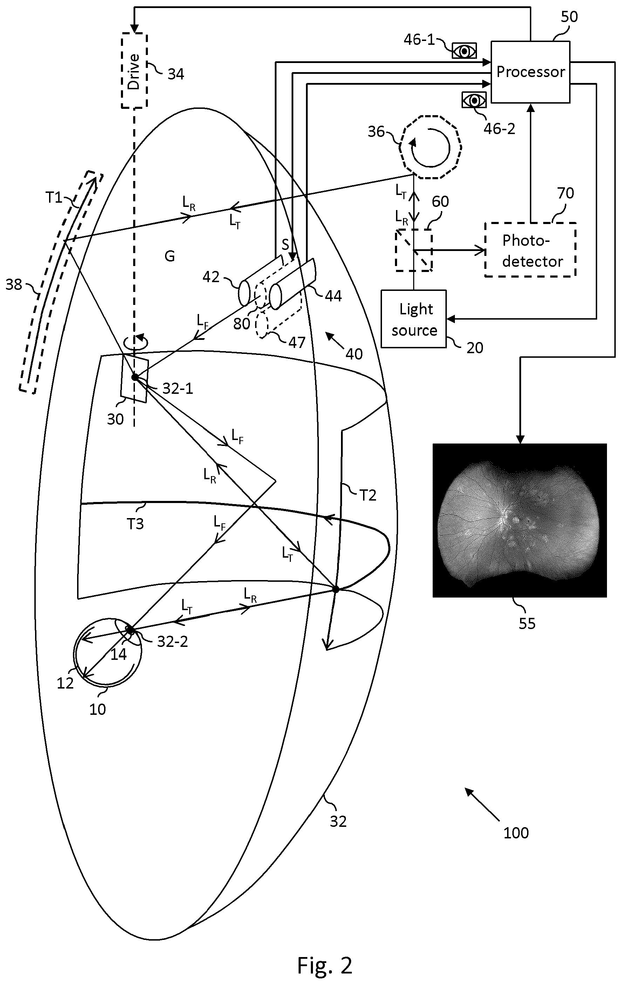

The ophthalmic imaging instrument 1 further comprises a patient alignment device 4 (also referred to herein as a patient alignment module (PAM) or a pupil alignment module), which is arranged to monitor a position of a pupil 14 of the eye 10 relative to the exit pupil E of the image acquisition apparatus 2. Although references are made herein to the exit pupil E, any other reference point of the image acquisition apparatus 2, in the vicinity of which the pupil 14 needs to be positioned for the acquisition of the image 5 of the fundus 12 of the eye 10, may be used instead. The patient alignment device 4 may measure the position of the pupil 14 along an optical axis (so-called "z-axis") of the image acquisition apparatus 2 which passes through the exit pupil E. The patient alignment device 4 may do this in a variety of different ways, and some example implementations are described herein, including an implementation based on stereoscopic range-finding, which is described below with reference to Figure 2.

The patient alignment device 4 is further arranged to compare the measured position with a range of distances R from the exit pupil E along the z-axis, within which range R the pupil 14 can be imaged by the image acquisition apparatus 2, to determine whether the pupil 14 is within the range of distances R from the exit pupil E. The range of distances R used in the comparison is defined by values stored in a memory of the patient alignment device 4, and can be adjusted in several different ways, as described in more detail below. This adjustability of the range of distances R used in the comparison may allow the sensitivity of the auto-capture mechanism described herein to displacements along the z-axis to be varied in dependence on the size of the pupil 14, specifically to require less precise alignment of the pupil 14 with the exit pupil E, which can be accepted in cases where the pupil 14 has been dilated (e.g. by application of eye drops containing a mydriatic) than in cases where the pupil 14 has not been dilated, thus allowing high quality fundus images to be captured more quickly and will less discomfort to dilated patients who are more sensitive to light.

In case the pupil 14 is determined to be outside the range of distances R, the patient alignment device 4 is arranged to generate, based on the monitored position, stimulus signals S (i.e. alerts in the form of lights of different colours, different sounds and/or different forms of haptic feedback, for example) for guiding the subject to vary a distance between the eye 10 and the image acquisition apparatus 2 to bring the pupil 14 towards and eventually into the range of distances R from the exit pupil E. In case the pupil 14 is determined to be within the range of distances R, the patient alignment device 4 is arranged to generate an indication I, which indicates that the pupil 14 is at a position suitable for acquiring the image 5 of the fundus 12.

The ophthalmic imaging instrument 1 further comprises a controller 6, which is arranged to control the image acquisition apparatus 2 automatically to acquire the image 5 of the fundus 12 in response to the patient alignment device 4 generating the indication I. The patient alignment device 4 and the controller 6 thus provide an auto-capture mechanism for automatically acquiring the image 5 of the fundus 12 when the pupil 14 comes sufficiently close to the exit pupil E for the image 5 to be acquired. This may be especially useful in the context of wide-field and ultra-widefield ophthalmic imaging instruments as described below, where insufficiently precise alignment of the patient's pupil with the instrument's exit pupil can lead to clipping of the fundus image by the pupil and thus less of the fundus being imaged than might be possible with a more precise pupil alignment.

Figure 2 is a schematic illustration of an ophthalmic imaging instrument 100, which is an example implementation of the ophthalmic imaging instrument 1 of Figure 1. The ophthalmic imaging instrument 100 is operable to image a portion of the fundus 12 by scanning a beam of light across the eye 10. The ophthalmic imaging instrument 100 may additionally be operable to image another portion of the eye 10, such as at least a part of the anterior segment of the eye 10.

The ophthalmic imaging instrument 100 may be a widefield (WF) ophthalmic imaging instrument, which is operable to acquire a WF image of the fundus 12. A WF image of the fundus 12 is defined as a single-capture image, centred on the fovea of the eye 10, which captures retinal anatomic features in the mid-periphery that are posterior to the vortex vein ampulla, in all four quadrants (i.e. superior, inferior, nasal and temporal) of the eye 10. A WF ophthalmic imaging instrument can thus acquire a single-capture image which covers a region of the retina extending from the fovea up to the posterior edge of vortex vein ampulla, and is defined to have a field of view (FoV) between 60 and 100 degrees. Here, the FoV is expressed in terms of the eye-angle, which is the angle subtended by the imaged retinal region at the spherical centre of the eye 10, i.e. the point of intersection between the vertical diameter of the eyeball and the visual axis.

The ophthalmic imaging instrument 100 may, as in the present example implementation, be provided in form of an ultra-widefield (UWF) ophthalmic imaging instrument, which has a larger FoV than a WF instrument and is operable to acquire an UWF image of the ocular fundus 12 covering a larger proportion of the ocular fundus 12. An UWF image of the ocular fundus 12 is defined as a single-capture image, centred on the fovea of the eye 10, which captures retinal anatomic features in the far periphery that are anterior to the vortex vein ampulla in all four quadrants. An UWF ophthalmic imaging instrument can thus acquire an UWF image being a single-capture image which covers a region of the retina extending from the fovea to the anterior edge of vortex vein ampulla and beyond to pars plana, and is defined to have a FoV (expressed in terms of the eye-angle) between 110 degrees and 220 degrees. By way of an example, the Optos Daytona^{™} is capable of UWF images (so -called Optomap^{™} images) covering up to 200 degrees of the fundus (or approximately 82% of the retina) in a single capture.

In the present example embodiment, the ophthalmic imaging instrument 100 comprises an UWF combined scanning laser ophthalmoscope (SLO) and optical coherence tomography (OCT) instrument, which is arranged to acquire OCT images of the ocular fundus 12 using well-known interferometric imaging techniques, as well as fundus images of one or more additional modalities. Examples of such additional imaging modalities include pseudo-colour imaging, fundus autofluorescence (FAF), fluorescein angiography (FA), and indocyanine green angiography (ICGA). By way of an example, the Optos Monaco^{™} is a combined SLO-OCT system capable of acquiring green or red (or combined green and red) laser views, and green laser autofluorescence views, as well as OCT views. However, the ophthalmic imaging instrument 100 need not be a combined SLO-OCT system, and may instead be an OCT imaging instrument operable to acquire OCT images only, or an UWF SLO operable in one or more of the aforementioned (or other) SLO imaging modalities but not in an OCT modality. The Optos Daytona^{™} is an example of such a multi-modal UWF SLO.

The image acquisition apparatus 2 may, as in the example implementation of Figure 2, comprise a light source 20 arranged to emit at least one beam (understood to be any directional projection) of light L_{T} for imaging the eye 10, a rotatable mirror 30 and an ellipsoidal mirror 32. The image acquisition apparatus 2 is arranged to employ these components to scan the at least one beam of light L_{T} across the eye 10, and collect and guide return light L_{R} from the region of the eye 10 illuminated by the beam of light L_{T}. The return light L_{R} may be a portion of the beam of light L_{T} which has been reflected or scattered from the eye 10, or it may be light stimulated by the beam of light L_{T}, as in the case of the FAF and ICGA imaging modalities, for example.

The patient alignment device 4 in the example implementation of Figure 2 comprises a stereo imaging apparatus 40 (described in more detail below) and a processor 50 to assist in bringing the eye 10 into a position at which it can be imaged by the ophthalmic imaging instrument 100. The stereo imaging apparatus 40 is arranged to acquire stereo images 46-1 and 46-2 of the pupil 14, and the processor 50 is arranged to process the stereo images 46-1 and 46-2 using any well-known stereoscopic distance calculation algorithm to monitor the position of the pupil 14 relative to the exit pupil E of the image acquisition apparatus 2. The processor 50 is further arranged to generate, based on the monitored position, the signals S for guiding the subject to vary the distance between the eye 10 and the image acquisition apparatus 2 to bring the pupil 14 towards the range of distances R from the exit pupil E if the pupil 14 is outside the range R. It should be noted, however, that the patient alignment device 4 need not rely on stereoscopic range-finding, as may instead employ any other means for determining the position of the pupil 14 relative to the exit pupil E of the image acquisition apparatus 2, such as a laser distance sensor or an arrangement as described in European patent application No. 24 165 816.0, which relies on an analysis of corneal specular reflections.

The processor 50 is further arranged to determine an indication of a size of the pupil 14 based on one or both of the stereo images 46-1 and 46-2 of the pupil 14, for example by using edge detection or other techniques (e.g. pixel value thresholding) to identity a representation of the pupil 14 in the image, and then determining, as a measure of the pupil size, a diameter, a circumference or an area of the pupil 14, for example. The processor 50 is further arranged to adjust, using the determined indication of the size of the pupil 14, the stored range of distances R, which is to be used in the comparison of the position of the pupil 14 with the range of distances R described above, such that the range R used in the comparison increases as the size of the pupil 14 increases. The stored range R may thus be changed from a first value (e.g. about 0.5 mm) required for the formation of an UWF image 55 with a small non-dilated pupil 14 (e.g. 2 mm in diameter), to a second value (e.g. about 2 mm) required for the formation of an UWF image 55 with a fully dilated pupil 14 (e.g. 8 mm in diameter). The processor 50 may switch between the first value and the second value for the range R by comparing the pupil size indicated by the determined indication with a predetermined threshold (e.g. 5 mm), and selecting the first value for the range R if the pupil size is below the predetermined threshold, and the second value for the range R if the pupil size is above the predetermined threshold. The processor 50 may alternatively vary the range R in one or more steps between the first value and the second value, or continuously (e.g. linearly) between the first value and the second value as a function of the pupil size. In the latter case, the range of distances R may be determined in dependence on the pupil size in any suitable way, for example using a look-up table or by evaluating a function of the pupil size.

The processor 50 in Figure 2 also provides an example implementation of the controller 6 of Figure 1. The controller 6 may, however, be implemented as a separate processor. The ophthalmic imaging instrument 100 may, as in the present example implementation, also include a beam splitter 60 and a photodetector 70. The beam splitter 60 is arranged to split off a portion of the return light L_{R} and direct the split-off portion of the return light L_{R} to the photodetector 70. An UWF image 55 of the eye 10 is acquired by the ophthalmic imaging instrument 100 (for example, by the processor 50) by processing the output of the photodetector 70 using well-known techniques.

The image acquisition apparatus 2 may also include a drive mechanism 34 comprising a galvanometer, for example, which is controlled by the processor 50 to cause the rotatable mirror 30 to scan the beam of light L_{T} by undergoing a predetermined rotational motion. This may, as in the present example embodiment, comprise a predetermined oscillatory rotational motion, wherein the rotatable mirror 30 rotates alternatively clockwise and anticlockwise by a predetermined angle (or, in other words, wherein the rotatable mirror 30 repeatedly rotates across an angular range defined about the mirror's axis of rotation, reversing its direction of rotation at each end of the angular range).

The light source 20 is, in general, arranged to generate light in one or more ranges of wavelength that are suitable for imaging the ocular fundus 12 (or other part of the eye 10, as the case may be), for example in the visible spectrum (e.g. red and green light) and/or the near-infrared spectrum. The light source 20 may, for example, comprise one or more laser diodes or one or more super-luminescent diodes (or a combination of one or more laser diodes and one or more super-luminescent diodes), and may also have one or more optical components, such as collimators, apertures and lenses, which are arranged to generate one or more light beams. The image acquisition apparatus 2 may be arranged to illuminate a region of the ocular fundus 12 with a (flying) spot of light or a line of light (in case of the ophthalmic imaging instrument 100 being a line-field (i.e. line-scanning) system) generated using a cylindrical lens or other well-known components or optical assemblies for generating line-field illumination. In example embodiments like the present, wherein the ophthalmic imaging instrument 100 is operable in an OCT imaging mode, a swept light source (in case of the ophthalmic imaging instrument 100 being a swept-source OCT (SS-OCT) system) or a broadband source (in case of the ophthalmic imaging instrument 100 being a spectral-domain OCT (SD-OCT) system) may be provided for OCT imaging.

The form of the photodetector 70 is not limited and the photodetector 70 may, for example, comprise a balanced photodetector arrangement comprising two reverse-biased photodiodes whose output photocurrents are subtracted from one another, the subtracted current signal being converted to a voltage detection signal by a transimpedance amplifier. In example embodiments where the ophthalmic imaging instrument 100 is provided in the form of a line-scan SLO, the photodetector 70 may comprise a one- or two-dimensional array of photo-sensing elements. In example embodiments like the present, where the ophthalmic imaging instrument 100 features an OCT imaging modality, a spectrometer (where a SD-OCT set-up is used) or a photodiode detector (where a SS-OCT set-up is used) may be provided to detect interference light from the sample arm and reference arm of the interferometer.

In the present example implementation, the image acquisition apparatus 2 is arranged to perform a two-dimensional point scan of the light L_{T} from the light source 20 across the region of the fundus 12 that is illuminated by the point-scan, and to collect light from the illuminated region during the point-scan. The image acquisition apparatus 2 is arranged to perform the two-dimensional point scan in part by the rotatable mirror 30 scanning the beam of light L_{T} across the fundus 12 via the ellipsoidal mirror 32. Furthermore, the image acquisition apparatus 2 further comprises a scanner 36 and a curved mirror 38, which are arranged to scan the beam of light L_{T} in a first direction across the ellipsoidal mirror 32 via the rotatable mirror 30. The scanner 36 may, as in the present example implementation, be provided in the form of a polygon scanner comprising a plurality (e.g. 16) facets arranged around the circumference of a wheel which is rotated at a high speed (typically over 30,000 revolutions per minute) to perform high-frequency repeat scans of the light beam L_{T}. However, the scanner 36 may be provided in other forms, such as a galvanometer scanner, a micro-electromechanical system (MEMS) scanning mirror or a resonant scanning mirror, for example, or may be replaced by a cylindrical lens or other means of generating a beam comprising a "fan" of light rays whose projection onto a flat surface forms a line of light in case the optical system is a line-field system. Where the ophthalmic imaging instrument 100 has an OCT imaging modality, as in the present example embodiment, a second galvanometer scanner (not shown in Fig. 2) may be provided to scan the OCT sample beam in the first direction across the ellipsoidal mirror 32, via the rotatable mirror 30.

The light beam L_{T} is reflected, in sequence, by the scanner 36, the curved mirror 38, the rotatable mirror 30 and the ellipsoidal mirror 32, before being incident on the fundus 12 via the pupil 14. Light from the illuminated region of the fundus 12 follows the same optical path through the image acquisition apparatus 2 as the light beam that had entered the optical system but does so in reverse order, and a part thereof is directed to the photodetector 70 by the beam splitter 60.

The two-dimensional point scan is performed by the scanner 36 scanning the light beam L_{T} in a first (e.g. vertical) direction across the region of the fundus 12, and by the rotatable mirror 30 rotating about its rotational axis to scan the light beam L_{T} in a second (e.g. horizontal) direction across the region of the fundus 12 (which second direction may, as in the present example embodiment, be orthogonal to the first direction). The ellipsoidal mirror 32 has a first focal point 32-1 and a conjugate second focal point 32-2 and may, as in the present example embodiment, take the form of a spheroidal mirror having an axis of rotational symmetry, specifically rotational symmetry about its major axis which passes through the focal points. More generally, any form of curved mirror having a curvature that enables it to transfer light rays incident on a first focal point of the curved mirror to a conjugate second focal point of the curved mirror may be employed in example embodiments as a generalisation of the ellipsoidal mirror 32. The ellipsoidal mirror 32 may be substantially spheroidal in some example embodiments, with some deviation in curvature from the spheroidal form that nevertheless allows the ophthalmic imaging instrument 100 to acquire WF or UWF images of the fundus 12. The rotational axis of the rotatable mirror 30 may, as in the present example embodiment, be parallel to the axis of circular symmetry of the spheroidal mirror. The length of the optical path between the first focal point 32-1, and points along a path T3 on the ellipsoidal mirror 32 followed by the beam of light L_{T} incident thereon as the beam is scanned by rotation of the rotatable mirror 30, is therefore constant. The scanning performed by the scanner 36 and the rotatable mirror 30 may be coordinated by the processor 50 or a scanning system controller (not shown) such that the beam of light L_{T} is scanned across the fundus 12 in accordance with a predefined scan pattern.

The curved mirror 38 (also referred to as a slit mirror) may be an ellipsoidal mirror, as in the present example embodiment. Each of the curved mirror 38 and the ellipsoidal mirror 32 thus has a respective first focal point and a respective conjugate second focal point. The scanner 36 is arranged to reflect the beam of light L_{T} at the first focal point of the curved mirror 38, such that a projection of the beam L_{T} on the curved mirror 38 follows the trajectory T1 shown in Figure 2. The curved mirror 38 reflects the beam of light L_{T} incident thereon to its second focal point, and the rotatable mirror 30 is arranged to reflect the beam of light L_{T} at the second focal point of the curved mirror 38 onto the ellipsoidal mirror 32. The rotatable mirror 30 is also arranged to reflect the beam L_{T} at the first focal point 32-1 of the ellipsoidal mirror 32, and the pupil 14 of the eye 10 is disposed at the second focal point 32-2 of the ellipsoidal mirror 32. As the beam of light L_{T} is scanned across the curved mirror 38, the beam L_{T} is reflected therefrom onto the ellipsoidal mirror 32 via the rotatable mirror 30, such that a projection of the beam L_{T} on the ellipsoidal mirror 32 follows the trajectory T2 shown in Figure 2 during performance of each so-called "vertical scan" along a so-called "vertical" (or "fast") scan direction across the ellipsoidal mirror 32. As the rotatable mirror 30 rotates, it causes the adjacent vertical scans to be displaced from one another along a so-called "horizontal" (or "slow") scan direction, such that points in the vertical scans having the same elevation are arranged along the path T3 illustrated in Figure 2.

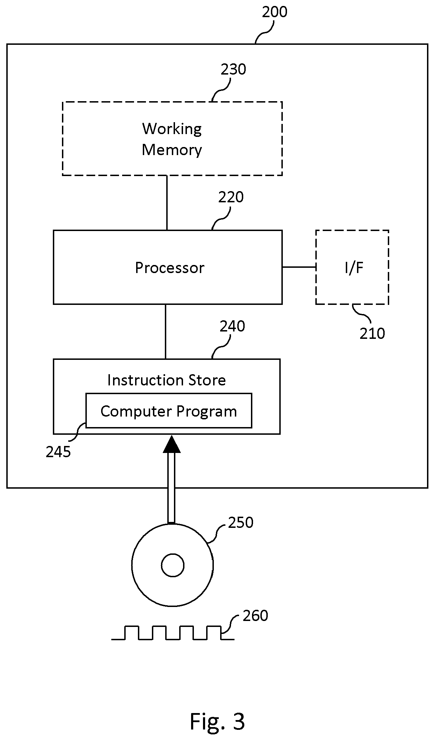

The processor 50 may be provided in any suitable form, for example as a processor 220 of a programmable signal processing hardware 200 of the kind illustrated schematically in Figure 3. The programmable signal processing hardware 200 comprises a communication interface (I/F) 210, for communicating control signals and/or data with the light source 20, the drive mechanism 34, the stereo imaging apparatus 40, the photodetector 70 and a fixation target light source, as described herein. The signal processing hardware 200 further comprises a processor 220 (e.g. a Central Processing Unit, CPU, and/or a Graphics Processing Unit, GPU), a working memory 230 (e.g. a random-access memory) and an instruction store 240 storing a computer program 245 comprising the computer-readable instructions which, when executed by the processor 220, cause the processor 220 to perform various functions of the processor 50 described herein.

The working memory 230 stores information used by the processor 220 during execution of the computer program 245. The instruction store 240 may comprise a ROM (e.g. in the form of an electrically erasable programmable read-only memory (EEPROM) or flash memory) which is pre-loaded with the computer-readable instructions. Alternatively, the instruction store 240 may comprise a RAM or similar type of memory, and the computer-readable instructions of the computer program 245 can be input thereto from a computer program product, such as a non-transitory, computer-readable storage medium 250 in the form of a CD-ROM, DVDROM, etc. or a computer-readable signal 260 carrying the computer-readable instructions. In any case, the computer program 245, when executed by the processor 220, causes the processor 220 to perform the functions of the processor 50 as described herein. More generally, the processor 50 of the present example embodiment may comprise one or more instances of the computer processor 220 and one or more instances of the memory 240 storing computer-readable instructions which, when executed by the computer processor(s) 220, cause the computer processor(s) 220 to perform the functions of the processor 50 as described herein. Where a plurality of processors 220 is provided, the processors 220 may communicate with each other via any kind of computer network.

It should be noted, however, that the processor 50 may alternatively be implemented in non-programmable hardware, such as an ASIC, an FPGA or other integrated circuit dedicated to performing the functions of the processor 50 described herein, or a combination of such non-programmable hardware and programmable hardware as described above with reference to Figure 3.

Referring again to Figure 2, the stereo imaging apparatus 40 comprises a first camera 42 and a second camera 44 that are arranged to acquire respective stereo images 46-1 and 46-2 of the pupil 14 and a surrounding portion of the eye 10 (including at least some of the iris and typically also some of the sclera) via the rotatable mirror 30 and the ellipsoidal mirror 32. The stereo imaging apparatus 40 may, as in the present example embodiment, also have an illumination source 47 which illuminates the eye 10 via the rotatable mirror 30 and the ellipsoidal mirror 32 with light whose reflection from the eye 10 is detected by the first camera 42 and the second camera 44 to acquire the respective stereo images 46-1 and 46-2 of the pupil 14. The illumination is typically in the infra-red (IR) band for patient comfort and to avoid pupil constriction, and may be provided by an illumination source 47 in the form of an IR light-emitting diode (LED), for example. The stereo cameras 42 and 44 may employ complementary metal oxide semiconductor (CMOS) sensors, and fast lenses of fixed focal length to compensate for the sensors' relatively low sensitivity to the IR illumination that is typically provided by the illumination source. The ophthalmic imaging instrument 100 may, as in the present example embodiment, further comprise (e.g. as part of the patient alignment device 4) a fixation target light source 80 comprising one or more light sources such as light-emitting diodes (LEDs), for example. The fixation target light source 80 is arranged to project fixation light L_{F} onto the fundus 12 via the rotatable mirror 30 and the ellipsoidal mirror 32. A projection of the fixation target light L_{F} on the fundus 12 has a predetermined shape chosen to assist the subject in maintaining a steady gaze direction, which may include one or more of cross hairs, a dot, a disc, and/or a circle or two or more concentric circles, for example. The colour of the fixation light L_{F} may be variable by the processor 50, as described below.

The processor 50 is arranged to operate in a patient alignment mode to control the drive mechanism 34 to rotate the rotatable mirror 30 to a predetermined orientation (if the rotatable mirror 30 is not already in that orientation), and to hold the rotatable mirror 30 stationary in the predetermined orientation while controlling the stereo imaging apparatus 40 to acquire the stereo images 46-1 and 46-2 of the pupil 14 at the same time (or with a small delay between the acquisitions of the two images that does not significantly affect the stereoscopic distance measurement) via the rotatable mirror 30 and the ellipsoidal mirror 32. The processor 50 controls the stereo imaging apparatus 40 to keep acquiring stereo images of the pupil 14 during operation in the patient alignment mode. The processor 50 is further configured to process the acquired stereo images to generate the signals S for bringing the pupil 14 within the (target) range of distances R from the exit pupil E which is suitable for acquiring an UWF image 55 of the fundus 12.

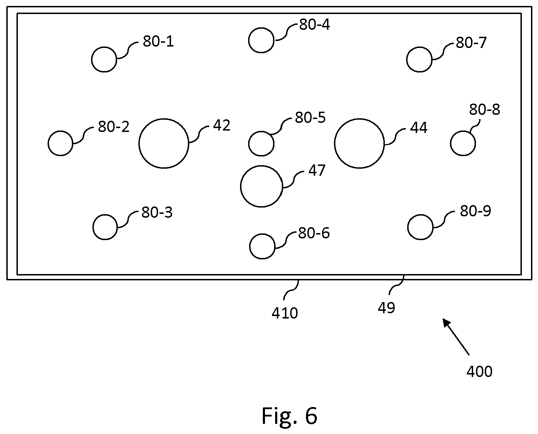

While the rotatable mirror 30 remains stationary in the predetermined orientation in the patient alignment mode, the processor 50 is also arranged to control the fixation target light source 80 to project the fixation light L_{F} onto the rotatable mirror 30, specifically onto the first focal point 32-1 of the ellipsoidal mirror 32. The fixation target light source 80 is disposed in relation to the rotatable mirror 30 whilst in the predetermined orientation such that the fixation light L_{F} is projected onto the fundus 12 via the rotatable mirror 30 and the ellipsoidal mirror 32 to fix the gaze direction of the eye 10 and thus keep the eye 10 steady while the pupil 14 is brought within the target range R suitable for acquiring the UWF image 55 of the fundus 12. The fixation target light source 80 may, as in the present example embodiment, be arranged to project the fixation light L_{F} onto the rotatable mirror 30 from a location such that, when the rotatable mirror 30 is in the predetermined orientation, the fixation light L_{F} is incident on the eye 10 in a direction for fixing the gaze direction of the eye 10 in a central gaze direction, which the subject adopts while looking straight ahead. However, as explained below with reference to Figure 6, the fixation target light source 80 may be configured to provide gaze fixation in gaze directions other than the central gaze direction.

In the patient alignment mode, the processor 50 may, as in the present example embodiment, process the stereo images 46-1 and 46-2 using any known stereoscopic ranging technique which uses the concept of parallax and triangulation to estimate distance, to determine an indication of a distance d between the exit pupil E of the ophthalmic imaging instrument 100 and the pupil 14 of the eye 10. For example, the processor 50 may determine the indication of the distance d by processing each image of the acquired stereo images 46-1 and 46-2 to locate a respective pupil centre of the pupil in the image (e.g. using edge detection to determine an outline of the pupil, and fitting a circle to the outline to find the circle centre), mapping the located pupil centres to a common image frame, determining a separation between the pupil centres in the common image frame, and determining the indication of the distance d using the determined separation, which is inversely related to the distance d. The processor 50 may then compare the determined indication of the distance d with pre-determined thresholds to determine whether the pupil 14 is too close to the exit pupil E, too far from exit pupil E, or within the predetermined range R of distances from the exit pupil E that is acceptable for image acquisition.

The processor 50 may, as in the present example embodiment, then generate the signals S to control the fixation target light source 80 to output fixation light of a colour that is indicative of the comparison result. For example, the fixation target light source 80 may be controlled by the processor 50 to emit fixation light of a first colour (e.g. blue) in case the distance between the exit pupil E of the ophthalmic imaging instrument 100 and the pupil 14 is greater than a threshold value demarcating the end of the range R and the pupil 14 is on a side of the range R further from the ophthalmic imaging instrument 100, to emit fixation light of a second, different colour (e.g. red) in case the distance between the exit pupil E and the pupil 14 is greater than the threshold value and the pupil 14 is on the other side of the range R which is nearer to the ophthalmic imaging instrument 100, and to emit fixation light of a yet further colour (e.g. green) in case the distance between the exit pupil E and the pupil 14 is smaller than the threshold value.

It should be noted, however, that the visual feedback provided to the subject to indicate whether the subject needs to move forward or backward to bring the pupil 14 into a position relative to the exit pupil E which is suitable for fundus imaging need not be conveyed via the fixation light L_{F}, and may be provided by any other light visible to the subject, for example background illumination to the fixation light L_{F}. Accordingly, the signals S may more generally comprise: a projection of light of a first colour (e.g. blue) onto the eye 10 in case the subject is to move the eye 10 towards the image acquisition apparatus 100 to bring the pupil 14 towards the range of distances R from the exit pupil E; a projection of light of a second colour (e.g. red) onto the eye 10 in case the subject is to move the eye 10 away from the image acquisition apparatus 100 to bring the pupil 14 towards the range of distances R from the exit pupil E; and a projection of light of a third colour (e.g. green) onto the eye 10 in case the pupil 14 is within the range of distances R from the exit pupil E suitable for acquiring the UWF image 55 of the fundus 12, wherein the first colour, the second colour and the third colour are different from one another.

Additionally or alternatively, the processor 50 may generate the signals S to control a speaker (not shown in Figure 2) to generate a sound (e.g. of a varying tone or spoken word(s)) which is indicative of the comparison result. For example, the speaker may be controlled by the processor 50 to generate a first (e.g. low-pitched) tone or a spoken indication, for example, in case the distance between the exit pupil E of the ophthalmic imaging instrument 100 and the pupil 14 is greater than the threshold value and the pupil 14 is on the side of the range R further from the ophthalmic imaging instrument 100, a different, second (e.g. high-pitched) tone or spoken indication in case the distance between the exit pupil E and the pupil 14 is greater than the threshold value and the pupil 14 is on the other side of the range R which is nearer to the ophthalmic imaging instrument 100, and a yet further (e.g. medium-pitched) tone or spoken indication in case the distance between the exit pupil E and the pupil 14 is smaller than the threshold value.

By the processing of each acquired pair of stereo images as described above, the subject may be guided to move the eye 10 backwards (away from the ophthalmic imaging instrument 100) or forwards (towards the ophthalmic imaging instrument 100), as appropriate, until the subject observes the fixation light to be green and/or hears the medium-pitched tone or spoken indication (as the case may be), informing the subject that the pupil 14 of the eye 10 is sufficiently close to the exit pupil E for the UWF imaging of the fundus 12 to begin.

After the distance between the exit pupil E and the pupil 14 has been determined to be smaller than the threshold value, the processor 50 is arranged to start operating in a fundus imaging mode to control the ophthalmic imaging instrument 100 to acquire the UWF image 55 of the fundus 12. The processor 50 does this by controlling the light source 20 to start emitting the beam of light L_{T}, and controlling the image acquisition apparatus 2 to acquire the UWF image 55 of the fundus 12 by controlling the scanner 36 and the rotatable mirror 30 to scan the beam of light L_{T} across the fundus 12 via the ellipsoidal mirror 32. The processor 50 preferably controls the fixation target light source 80 to stop emitting the fixation light L_{F} (before or after the light source 20 starts emitting the beam of light L_{T}) to reduce artifacts and otherwise improve the quality of the UWF image 55. To reduce the risk of the eye 10 becoming misaligned with the ophthalmic imaging instrument 100, the processor 50 may, as in the present example embodiment, begin to operate in the fundus imaging mode automatically in response to determining that the distance between the exit pupil E of the ophthalmic imaging instrument 100 and the pupil 14 is smaller than the threshold value. However, in other example embodiments, this switch in the mode of operation may be instructed by an operator of the ophthalmic imaging instrument 100 in reaction to visual, audio and/or tactile feedback based on the signals S, for example by the operator using a user clicking a mouse button or pressing a key on a computer keyboard communicatively coupled to the processor 50 (as examples of a user interface which may be employed for this purpose).

The stereo imaging apparatus 40 may, as in the present example embodiment, be located within a region G between the rotatable mirror 30 and the ellipsoidal mirror 32 which is not traversed by the beam of light L_{T} during acquisition of the UWF image 55. The fixation target light source 80 may, as in the present example embodiment, also be located within the region G. The stereo imaging apparatus 40 and fixation target light source 80 may thus be accommodated in a region within the bowl of the ellipsoidal mirror 32 which is not used for beam propagation. This arrangement may advantageously allow the ophthalmic imaging instrument 100 to be made more compact, by placing the stereo imaging apparatus 40 and the fixation target light source 80 in an otherwise unused region of the ophthalmic imaging instrument 100.

Furthermore, accommodating the stereo imaging apparatus 40 and the fixation target light source 80 in the region G allows for the option of orientating the rotatable mirror 30 in the patient alignment mode (i.e. choosing the predetermined orientation mentioned above) such that the rotatable mirror 30 starts rotating from the predetermined orientation as soon as the UWF ophthalmic imaging instrument 100 starts acquiring the UWF image 55 during operation of the processor 50 in the fundus imaging mode. A delay in the start of image acquisition, which would be caused by a rotation of the rotatable mirror 30 from the predetermined orientation used in the patient alignment mode to a (different) start orientation for image acquisition, may therefore be avoided, and the risk of the gaze direction of the eye 10 changing thus reduced. However, if this advantage is outweighed by the need to provide easy access to the stereo imaging apparatus 40 or the fixation target light source 80 for maintenance and/or adjustment, for example, the stereo imaging apparatus 40 and the fixation target light source 80 may instead be located outside the bowl of the ellipsoidal mirror 32 in some example embodiments, with the predetermined orientation of the rotatable mirror 30 used in the patient alignment mode being set to still allow the stereo imaging apparatus 40 to acquire the stereo images 46-1 and 46-2 of the pupil 14 via the rotatable mirror 30 and the ellipsoidal mirror 32 while the eye 10 remains fixated. For example, the stereo imaging apparatus 40 and the fixation target light source 80 may be located above the rim of the ellipsoidal mirror (similarly to the curved mirror 38 in Figure 1, for example), to each have a line of sight to the rotatable mirror 30. Alternatively, the stereo imaging apparatus 40 and the fixation target light source 80 may be located on the non-reflecting side of the ellipsoidal mirror 32, with one or more holes being provided in the ellipsoidal mirror 32 to allow light to pass through the ellipsoidal mirror 32 to the stereo imaging apparatus 40 and from the fixation target light source 80.

Regardless of whether the rotatable mirror 30 is orientated in the patient alignment mode to allow the rotatable mirror 30 to start rotating from the predetermined orientation when the UWF ophthalmic imaging instrument 100 starts acquiring the UWF image 55, it may be advantageous to locate the stereo imaging apparatus 40 between the rotatable mirror 30 and the ellipsoidal mirror 32 at a location which minimises a deviation from circularity of respective representations of the pupil 14 in the stereo images 46-1 and 46-2 of the pupil 14 acquired by the stereo imaging apparatus 40 when the rotatable mirror 30 is in the predetermined orientation. Such a location of the stereo imaging apparatus 40 to bring its viewpoint to be as close as possible to a central gaze direction of the eye 10, and thus allow the pupil 14 in the images 46-1 and 46-2 to be as circular as possible, may facilitate the process of locating the pupil centre and therefore an accurate calculation of the distance between the pupil 14 and the exit pupil E.

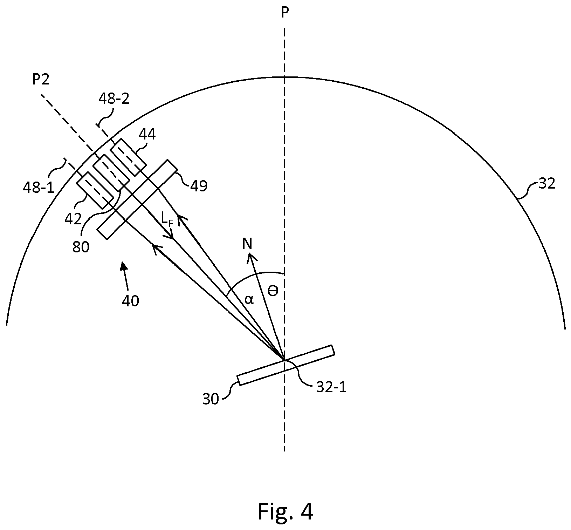

By way of an example, the ellipsoidal mirror 32 of the present example embodiment has a plane of symmetry P comprising the first focal point 32-1 and the second focal point 32-2, and a normal direction N of the rotatable mirror 30 at the first focal point 32-1 subtends a predetermined angle θ relative to the plane of symmetry P when the rotatable mirror 30 is in the predetermined orientation, as illustrated in Figure 4. For clarity, the sizes of angle θ and angle α discussed below have been exaggerated in Figure 4. The first camera 42 and the second camera 44 are arranged symmetrically about a second plane P2, which passes through the first focal point 32-1 and the second focal point 32-2. The first camera 42 and the second camera 44 may, as in the present example embodiment, comprise respective camera lenses having respective optical axes 48-1 and 48-2 that are parallel to each other and arranged equidistantly from, and on opposite sides of, the second plane P2. However, in other example embodiments, the optical axes 48-1 and 48-2 may both be within the second plane P2. Where the normal direction N of the rotatable mirror 30 and the second plane P2 subtend an angle α at the first focal point 32-1 which is less than two times as large as the predetermined angle θ, the deviation from circularity of respective representations of the pupil 14 in the stereo images 46-1 and 46-2 of the pupil 14 acquired using this arrangement was found to be small enough to allow satisfactory pupil alignment to be achieved.

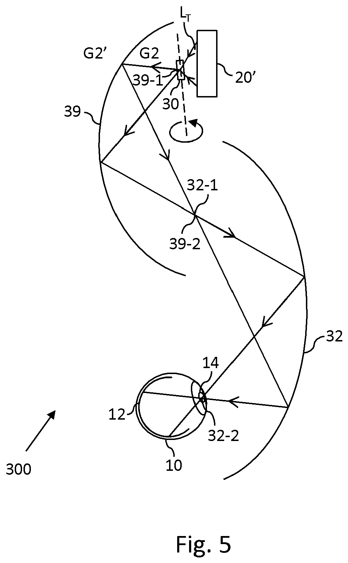

It should be noted that, although the rotatable mirror 30 is provided to reflect the beam of light L_{T} across the ellipsoidal mirror 32 at the first focal point 32-1 of the ellipsoidal mirror 32, the optical system of the ophthalmic imaging instrument 100 may be arranged such that the rotatable mirror 30 scans the beam L_{T} via the first focal point 32-1 in another way. Figure 5 is a schematic illustration of a line-scan UWF ophthalmic imaging instrument 300 according to a variant of the example implementation of Figure 2 which comprises, in addition to the ellipsoidal mirror 32, a second ellipsoidal mirror 39, via which the rotatable mirror 30 is arranged to scan a beam of light L_{T}' in the form of a line of light across the fundus 12. In this case, the beam of light L_{T}' has a projection onto a plane normal to its direction of propagation (i.e. a cross-section) which is a line, and may be generated from a beam of light having a cross-section of a spot (as generated by the light source 20) using a cylindrical lens, or may be generated using another optical arrangement for generating line-field illumination known to those versed in the art (e.g. a back-lit slit aperture). As illustrated in Figure 5, the second ellipsoidal mirror 39 has a first focal point 39-1 and a second focal point 39-2, with the rotatable mirror 30 being arranged to reflect the beam of light L_{T}' at the first focal point 39-1 of the second ellipsoidal mirror 39. The second focal point 39-2 of the second ellipsoidal mirror 39 coincides with the first focal point 32-1 of the ellipsoidal mirror 32.

The light source 20', which generates the line-field illumination L_{T}', is shown in Figure 5, and this is provided instead of the light source 20, the scanner 36 and the curved mirror 38 of the example embodiment of Figure 2. The line-scan UWF ophthalmic imaging instrument 300 includes the remaining components of the UWF ophthalmic imaging instrument 100, although these are not shown in Figure 5 to more clearly illustrate the different scan transfer arrangement comprising the two ellipsoidal mirrors 32 and 39. The line-scan UWF ophthalmic imaging instrument 300 also has a beam splitter to direct the return light from the fundus 12 to a photodetector for detecting the return line-field illumination, although this has similarly been omitted from Figure 5 for clarity.

In the line-scan UWF ophthalmic imaging instrument 300, the stereo imaging apparatus 40 and the fixation target light source 80 (where provided) may be accommodated in a region G2 (between the second ellipsoidal mirror 39 and the rotatable mirror 30) not traversed by the beam of light L_{T}'. The stereo imaging apparatus 40 and the fixation target light source 80 (where provided) may be arranged in relation to the rotatable mirror 30 and configured to operate as described above, although with the light propagating to the stereo imaging apparatus 40 from the eye 10, and the fixation light L_{F} propagating from the fixation target light source 80 to the eye 10, doing so via the second ellipsoidal mirror 39 as well as the ellipsoidal mirror 32.

Alternatively, if there is insufficient space within the bowl of the second ellipsoidal mirror 39, one or both of the stereo imaging apparatus 40 and the fixation target light source 80 may be located in a region G2' on the non-reflecting side of the second ellipsoidal mirror 39, with one or more holes being provided in the second ellipsoidal mirror 39 to allow light to pass through the second ellipsoidal mirror 39 to the stereo imaging apparatus 40 and from the fixation target light source 80.

Regardless of whether the stereo imaging apparatus 40 is accommodated within the region G (or region G2 in the variant of Figure 5), the stereo imaging apparatus 40 may, as in the present example embodiment, further comprise a Fresnel lens 49 disposed between the rotatable mirror 30 and both the first camera 42 and the second camera 44. In the example embodiment of Figure 2, the Fresnel lens 49 is arranged to refract light from the eye 10, which has been reflected by the rotatable mirror 30 at the first focal point 32-1 of the ellipsoidal mirror 32, to propagate along a first optical axis 48-1 of the first camera 42, and along a second optical axis 48-2 of the second camera 44. The Fresnel lens 49 allows the first camera 42 and the second camera 44 to be easily mounted on a supporting circuit board or other flat substrate, with their respective optical axes being normal to the surface of the substrate. The time-consuming process of carefully aligning the optical axes of the first camera 42 and the second camera 44 to pass through the first focal point 32-1 of the ellipsoidal mirror 32, which may otherwise need to be performed to improve the stereoscopic ranging, may be avoided, and the manufacture of the stereo imaging apparatus 40 consequently made simpler and faster. Similarly, in the variant described above with reference to Figure 5, the Fresnel lens 49 may be disposed between the rotatable mirror 30 and both the first camera 42 and the second camera 44 that are located in the region G2, and arranged to refract light from the eye 10, which has been reflected by the rotatable mirror 30 at the first focal point 39-1 of the second ellipsoidal mirror 39, to propagate along a first optical axis 48-1 of the first camera 42, and along a second optical axis 48-2 of the second camera 44.

Although the fixation target light source 80 is operable to project the fixation light L_{F} onto the rotatable mirror 30 from a single location for central gaze fixation in the example embodiment and the variant thereof described above, the fixation target light source 80 may alternatively be configured to project the fixation light L_{F} onto the rotatable mirror 30 from a selected location of a plurality of locations on the fixation target light source 80 that are arranged in different respective directions from the first focal point 32-1 of the ellipsoidal mirror 32 (or from the first focal point 39-1 of the second ellipsoidal mirror 39 in the variant of Figure 5).

Figure 6 is a schematic illustration of a patient alignment device 400 comprising the stereo imaging system 40 and a modified version of the fixation target light source 80 included in the example implementation of Figure 2, when viewed from the rotatable mirror 30. As shown in Figure 6, the patient alignment device 400 comprises a flat substrate 410, onto which are mounted the first camera 42 and the second camera 44, as well as an IR illumination source 47 for illuminating the eye 10 so that it can be imaged by the stereo cameras 42 and 44. The fixation target light source 80 comprises a plurality of separate fixation light sources in the form of LEDs, labelled 80-1 to 80-9 in Figure 5. Each of these fixation target light sources may generate visible light of at least three colours, for example red, green and blue. In the example of Figure 6, the LEDs 80-1 to 80-9 are RGB LEDs, each capable of emitting red, green or blue light under the control of the processor 50. Although there are nine such light sources which are arranged as shown in the example of Figure 6, neither their number not arrangement is so limited. The patient alignment device 400 may, as in the present example, also include the Fresnel lens 49 mentioned above, which is overlaid on the stereo cameras 42 and 44, the IR illumination source 47 and the LEDs 80-1 to 80-9 so that it is disposed between these components and the rotatable mirror 30.

The LEDs 80-1 to 80-9 are provided at different respective locations that are arranged in different respective directions from the first focal point 32-1 in the example implementation of Figure 2 (or the first focal point 39-1 of the second ellipsoidal mirror 39 in the variant of Figure 5) so that the eye 10 can be steered in different directions by fixating on the light that is relayed to the eye 10 via the rotatable mirror 30 and the ellipsoidal mirror 32 (and the second ellipsoidal mirror 39 in the variant of Figure 5) from a selected one of the LEDs. In the example of Figure 6, LED 80-5 is arranged to provide central gaze fixation, while the remaining LEDs are arranged to guide the patient to look left, right, straight up, up and to the left, up and to the right, straight down, down and to the left, and down and to the right.

It should be noted, however, that the fixation target light source 80 may be arranged in other ways to project the fixation light L_{F} onto the rotatable mirror 30 from a selected location of a plurality of locations on the fixation target light source 80 that are arranged in different respective directions from the first focal point 32-1 of the ellipsoidal mirror 32 (or from the first focal point 39-1 of the second ellipsoidal mirror 39 in the variant of Figure 5). For example, the fixation target light source 80 may be provided in the alternative form of at least one display screen (e.g. a liquid crystal display (LCD), an LED display screen of an organic LED (OLED) display screen) which is controllable by the processor 50 to project the fixation light L_{F} from a selected portion of a plurality of separated portions on the display screen, and in some cases in a selected colour of a plurality of available colours, depending on the displacement of the pupil 14 from the exit pupil E. The background colour of the display screen(s) may be varied as described above to indicate whether the subject needs to move the eye 10 forward or backward (or not at all) for the pupil 14 to be within the range of distances R. As another example, the fixation target light source 80 may be provided in the form of an arrangement of optical fibres, which may emerge from the substrate 410 at the same locations as the locations of the LEDs 80-1 to 80-9 and project light being guided therethrough towards the rotatable mirror 30, wherein optical switches or other means controllable by the processor 50 are provided to allow fixation light L_{F} from a selected optical fibre to be incident on the rotatable mirror 30.

In the patient alignment mode, the processor 50 is arranged to select a gaze direction from a plurality of different gaze directions, in which gaze direction a gaze direction of the eye 10 is to be fixed, and determine, using the selected gaze direction, a corresponding location of the plurality of locations, from which location the fixation target light source 80 is to project the fixation light L_{F} onto the rotatable mirror 30. In the example of Figure 6, the processor 50 may selected one of the LEDs 80-1 to 80-9, which corresponds to the selected gaze direction. The processor 50 may then control the fixation target light source 80 to project the fixation light L_{F} onto the rotatable mirror 30 from the determined location to fix the gaze direction of the eye 10 in the selected gaze direction, for example by driving only the selected LED to emit the target fixation light L_{F} of the appropriate colour.

The plurality of different gaze directions may comprise a central gaze direction, and the plurality of locations on the fixation target light source 80 may comprises a central location (i.e. the location of LED 80-5 in the example of Figure 6) for fixing the gaze direction of the eye 10 in the central gaze direction when the fixation light L_{F} is projected onto the fundus 12 from the central location via the rotatable mirror 30 in the predetermined orientation and via the ellipsoidal mirror 32. For each location of the locations on the fixation target light source 80 other than the central location, the Fresnel lens 49 is arranged to refract the fixation light L_{F}, when emitted from the location, to propagate towards a common point on the rotatable mirror 30. This common point corresponds to the first focal point of the ellipsoidal mirror 32 in the above example embodiment, and the first focal point 39-1 of the second ellipsoidal mirror 39 in the variant thereof described with reference to Figure 5. Accordingly, the Fresnel lens 49 can provide the necessary focussing of both the return light from the eye 10 used to form the stereo images 46-1 and 46-2 and the fixation light L_{F} used to fix the gaze direction of the eye 10, thereby avoiding the need to provide separate optics for the stereo imaging apparatus 40 and the fixation target light source 80.

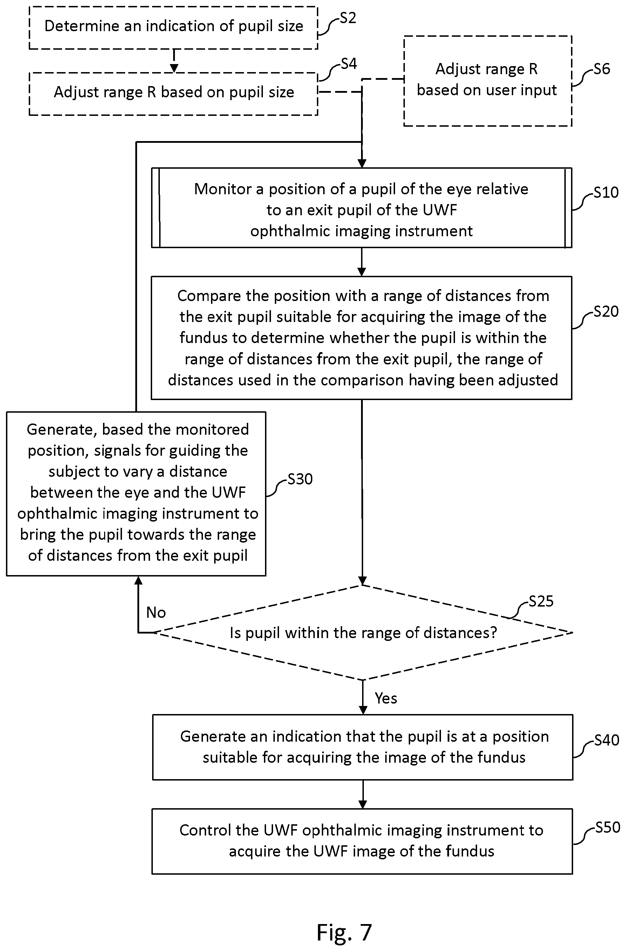

Figure 7 is a flow diagram summarising the operations performed by the processor 50 as described above to control the ophthalmic imaging instrument 100 to acquire an UWF image 55 of the fundus 12. For clarity, some actions and processes that may be taken/performed in the context of these operations as described above will not be described again here but are understood to form optional features of the operations described in the following with reference to Figure 7. The processor 50 may similarly control the ophthalmic imaging instrument 300 to acquire an UWF image 55 of the fundus 12.

When operating in the patient alignment mode to generate signals S to bring the pupil 14 of the eye 10 within the target range R for acquiring the UWF image 55, the processor 50 may firstly control the rotatable mirror 30 to be stationary in a predetermined orientation that allows the stereo imaging apparatus 40 to image the pupil 14 via the rotatable mirror 30 and the ellipsoidal mirror 32. The processer 50 may then control the illumination source of the stereo imaging apparatus 40 to turn on, thereby illuminating the eye 10 with IR light via the rotatable mirror 30 and the ellipsoidal mirror 32 (and via the second ellipsoidal mirror 39 in the variant of Figure 5).

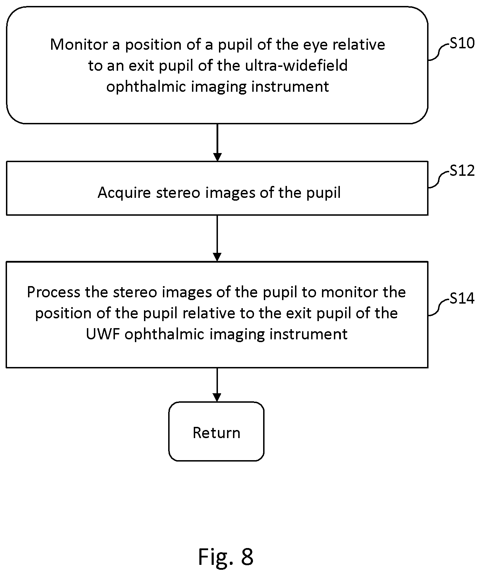

In process S10 of Figure 7, the processor 50 monitors a position of the pupil 14 relative to an exit pupil E of the UWF ophthalmic imaging instrument 100. The processor 50 may, as in the present example embodiment, monitor this position by the processes shown in the flow chart of Figure 8, specifically by acquiring stereo images 46-1 and 46-2 of the pupil 14 in process S12, and processing the stereo images 46-1 and 46-2 in process S14, for example as described above, to monitor the position of the pupil 14 relative to the exit pupil E of the UWF ophthalmic imaging instrument 100.

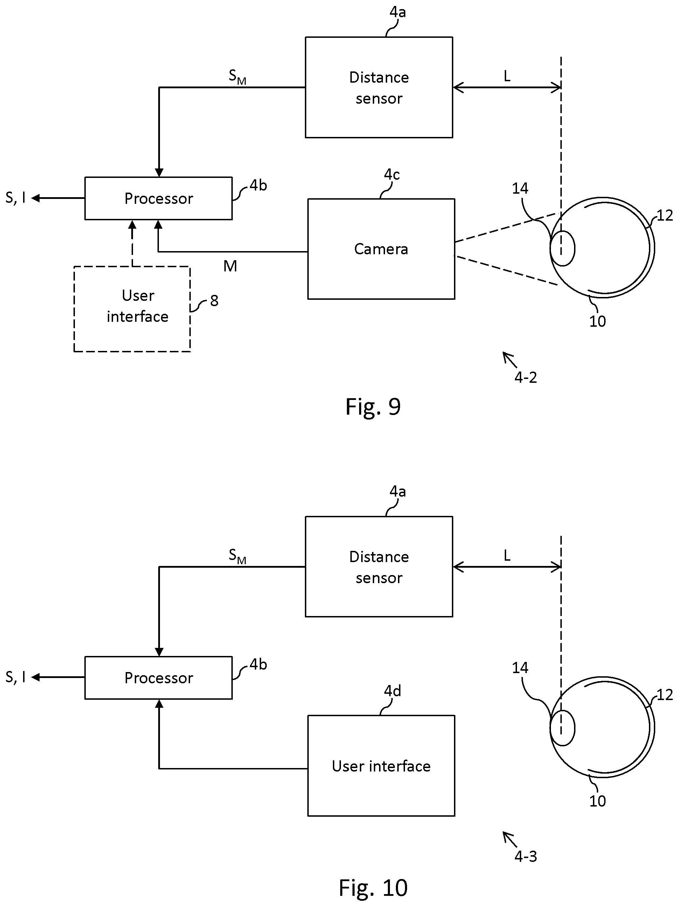

Referring again to Figure 7, in process S20, the processor 50 compares the position with a range of distances R from the exit pupil E suitable for acquiring the UWF image 55 of the fundus 12 to determine whether the pupil 14 is within the range of distances R from the exit pupil E, the range of distances R used in the comparison having previously been adjusted. The range of distances R used by the processor 50 in the comparison of S20 may, for example, have been adjusted in optional processes S2 and S4 shown in Figure 7, specifically by the processor 50 determining (in process S2) an indication of a size of the pupil 14 (e.g. a diameter, a circumference or an area of the pupil 14) based on at least one of the stereo images 46-1 and 46-2 of the pupil 14, and adjusting (in process S4) the range of distances R used in the comparison of S20, based on the determined indication of the size of the pupil 14, such that the range of distances R used in the comparison S20 increases as the size of the pupil 14 increases. Alternatively, where the patient alignment device comprises a single (non-stereo) camera 4c, as described herein with reference to Figure 9, the processor 50 may determine an indication of a size of the pupil 14 based on an image of the pupil 14 captured by the camera 4c (rather than one or both of the stereo images 46-1 and 46-2) in S2 of Figure 7. The adjustment of the range R is not limited to these examples, however, and may be done in other ways, for example by providing the patient alignment device with a user interface 4d, as described herein with reference to Figure 10, and configuring the processor 50 to perform, instead of processes S2 and S4, process S6 in Figure 7, where the processor 50 adjusts the range of distances R used in the comparison in accordance with a user input provided via the user interface 4d. In this case, the user has knowledge of the state of pupil dilation, and can manually adjust the range of distances R as required.

In case the pupil 14 is determined in S20 to be outside the range of distances R ("No" at S25), then in process S30 of Figure 7, the processor 50 generates, based on the monitored position, signals S for guiding the subject to vary a distance between the eye 10 and the UWF ophthalmic imaging instrument 100 to bring the pupil 14 towards the range of distances R from the exit pupil E. These signals S may comprise: a projection of light of a first colour onto the eye 10 in case the subject is to move the eye 10 towards the UWF ophthalmic imaging instrument 100 to bring the pupil 14 towards the range of distances R from the exit pupil E; a projection of light of a second colour onto the eye 10 in case the subject is to move the eye 10 away from the UWF ophthalmic imaging instrument 100 to bring the pupil 14 towards the range of distances R; and a projection of light of a third colour onto the eye 10 in case the pupil 10 is within the range of distances R from the exit pupil E suitable for acquiring the UWF image 55 of the fundus 12, wherein the first colour, the second colour and the third colour are different from one another.

In case the pupil 14 is determined in S20 to be within the range of distances R ("Yes" at S25), then in process S40 of Figure 7, the processor 50 generates an indication I that the pupil 14 is at a position suitable for acquiring the UWF image 55. In response to the indication I being generated in process S40, the processor 50 automatically controls (in process S50 of Figure 7) the UWF imaging instrument 100 to acquire the UWF image 55 of the fundus 12. The processor 50 may control the light source 20 to emit the beam of light L_{T}, and control the UWF ophthalmic imaging instrument 100 to acquire the UWF image 55 by rotating the rotatable mirror 30 to scan the beam of light L_{T} across the fundus 12 via the ellipsoidal mirror 32 (and via the second ellipsoidal mirror 39 in the variant of Figure 4). Prior to the acquisition of the UWF image 55, the processor 50 preferably turns off the fixation target light source 80 and the IR illumination source 47.

In the example embodiments described above, the patient alignment device 4 comprises a stereo imaging apparatus 40, and a processor 50 arranged to determine an indication of a size of the pupil 14 based on one or both of the stereo images 46-1 and 46-2 of the pupil 14 captured by the stereo imaging apparatus 40, and to adjust the range of distances R used in the comparison, based on the determined indication of the size of the pupil 14. However, the patient alignment device is not so limited, and may be configured to adjust the range of distances R used in the comparison in other ways.

Figure 9 is a schematic illustration of a variant patient alignment device 4-2, which comprises a distance sensor 4a arranged to measure a distance L to the eye 10 and generate a measurement signal S_{M} indicative of the measured distance L. The distance sensor 4a may, for example, comprise a laser distance sensor, which may be arranged to measure the distance to the pupil 14 using laser triangulation, for example. The patient alignment device 4-2 also has a processor 4b arranged to monitor the position of the pupil 14 relative to the exit pupil E of the image acquisition apparatus 2 based on the measurement signal S_{M}. The patient alignment device 4-2 further comprises a camera 4c arranged to capture an image M of the pupil 14, and the processor 4b is further arranged to determine an indication of a size of the pupil 14 based on the captured image M of the pupil 14 (similarly to the processor 50 based on the stereo image 46-1 and/or stereo image 46-2), and to adjust the range of distances R used in the comparison based on the determined indication of the size of the pupil 14 such that the range of distances R used in the comparison increases as the size of the pupil 14 increases, as described above.

Figure 10 is a schematic illustration of another variant patient alignment device 4-3, wherein the patient alignment device 4-3 comprises a user interface 4d by which the range of distances R used in the comparison is adjustable by a user of the UWF ophthalmic imaging instrument 100. The user interface 4d may be included in any of the example embodiments and variants thereof described above, to allow the user to override the automatic adjustment of the range R being performed, if required.

Some aspects of the examples presented herein, such as the functions of the processor 50, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein.

A computer program product may be provided in the form of a storage medium or media, instruction store(s), or storage device(s), having instructions stored thereon or therein which can be used to control, or cause, a computer or computer processor to perform any of the procedures of the example embodiments described herein.

## Claims

1. An ultra-widefield ophthalmic imaging instrument (1; 100; 300) comprising:
an image acquisition apparatus (2) arranged to acquire an ultra-widefield image (55) of a fundus (12) of an eye (10) of a subject via an exit pupil (E) of the image acquisition apparatus (2);
a patient alignment device (4; 4-2; 4-3) comprising:
a stereo imaging apparatus (40) arranged to acquire stereo images (46-1; 46-2) of the pupil (14); and
a processor (50) arranged to process the stereo images (46-1; 46-2) to monitor a position of a pupil (14) of the eye (10) relative to the exit pupil (E) of the image acquisition apparatus (2), and compare the position with a range of distances (R) from the exit pupil (E) suitable for acquiring the image (55) of the fundus (12) to determine whether the pupil (14) is within the range of distances (R) from the exit pupil (E), the range of distances (R) used in the comparison being adjustable, wherein
the processor (50) is further arranged to determine an indication of a size of the pupil (14) based on at least one of the stereo images (46-1; 46-2) of the pupil (14), and to adjust the range of distances (R) used in the comparison of the position with the range of distances (R), based on the determined indication of the size of the pupil (14), such that the range of distances (R) used in the comparison increases as the size of the pupil (14) increases,
in case the pupil (14) is determined to be outside the range of distances (R), the patient alignment device (4; 4-2; 4-3) is arranged to generate, based on the monitored position, signals (S) for guiding the subject to vary a distance between the eye (10) and the image acquisition apparatus (2) to bring the pupil (14) towards the range of distances (R) from the exit pupil (E), and
in case the pupil (14) is determined to be within the range of distances (R), the patient alignment device (4; 4-2; 4-3) is arranged to generate an indication (I) that the pupil (14) is at a position suitable for acquiring the image (55) of the fundus (12); and
a controller (6) arranged to control the image acquisition apparatus (2) automatically to acquire the ultra-widefield image (55) of the fundus (12) in response to the patient alignment device (4; 4-2; 4-3) generating the indication (I).

2. The ultra-widefield ophthalmic imaging instrument (100; 300) according to Claim 1, wherein the image acquisition apparatus (2) is arranged to acquire the ultra-widefield image (55) of the fundus (12) by scanning a beam of light (L_{T}) across the fundus (12), and comprises:
a light source (20; 20') arranged to emit the beam of light (L_{T}; L_{T}');
a rotatable mirror (30) arranged to scan the beam of light (L_{T}; L_{T}') across the fundus (12); and
an ellipsoidal mirror (32) via which the rotatable mirror (30) is arranged to scan the beam of light (L_{T}; L_{T}') across the fundus (12), the ellipsoidal mirror (32) having a first focal point (32-1) and a second focal point (32-2), wherein the rotatable mirror (30) is arranged to scan the beam of light (L_{T}; L_{T}') across the ellipsoidal mirror (32) via the first focal point (32-1), and the pupil (14) of the eye (10) is positioned at the second focal point (32-2) during use of the ultra-widefield ophthalmic imaging instrument (100),
wherein the controller (6) is arranged to operate in a patient alignment mode to cause the patient alignment device (4) to monitor the position of the pupil (14) relative to the exit pupil (E) of the image acquisition apparatus (2), and to operate in a fundus imaging mode to control the image acquisition apparatus (2) to acquire the ultra-widefield image (55) of the fundus (12), wherein
the stereo imaging apparatus (40) is arranged to acquire the stereo images (46-1; 46-2) of the pupil (14) via the rotatable mirror (30) and the ellipsoidal mirror (32),
in the patient alignment mode, the controller (6) is arranged to:
control the rotatable mirror (30) to be stationary in a predetermined orientation; and
control the stereo imaging apparatus (40) to acquire the stereo images (46-1; 46-2) of the pupil (14) via the rotatable mirror (30) and the ellipsoidal mirror (32) while the rotatable mirror (30) is stationary in the predetermined orientation, and
in the fundus imaging mode, the controller (6) is arranged to:
control the light source (20; 20') to emit the beam of light (L_{T}; L_{T}'); and
control the image acquisition apparatus (2) to acquire the ultra-widefield image (55) of the fundus (12) by controlling the rotatable mirror (30) to scan the beam of light (L_{T}; L_{T}') across the fundus (12) via the ellipsoidal mirror (32).

3. An ultra-widefield ophthalmic imaging instrument (1; 100; 300) comprising:
an image acquisition apparatus (2) arranged to acquire an ultra-widefield image (55) of a fundus (12) of an eye (10) of a subject via an exit pupil (E) of the image acquisition apparatus (2);
a patient alignment device (4; 4-2; 4-3) comprising:
a distance sensor (4a) arranged to measure a distance (L) to the eye (10) and generate a measurement signal (S_{M}) indicative of the measured distance (L), the patient alignment device (4-2) being arranged to monitor a position of a pupil (14) of the eye (10) relative to the exit pupil (E) of the image acquisition apparatus (2) based on the measurement signal (S_{M}), and compare the position with a range of distances (R) from the exit pupil (E) suitable for acquiring the image (55) of the fundus (12) to determine whether the pupil (14) is within the range of distances (R) from the exit pupil (E), the range of distances (R) used in the comparison being adjustable; and
a camera (4c) arranged to capture an image (M) of the pupil (14), the patient alignment device (4-2) being further arranged to determine an indication of a size of the pupil (14) based on the captured image (M) of the pupil (14), and adjust the range of distances (R) used in the comparison of the position with the range of distances (R), based on the determined indication of the size of the pupil (14), such that the range of distances (R) used in the comparison increases as the size of the pupil (14) increases, wherein
in case the pupil (14) is determined to be outside the range of distances (R), the patient alignment device (4; 4-2; 4-3) is arranged to generate, based on the monitored position, signals (S) for guiding the subject to vary a distance between the eye (10) and the image acquisition apparatus (2) to bring the pupil (14) towards the range of distances (R) from the exit pupil (E), and
in case the pupil (14) is determined to be within the range of distances (R), the patient alignment device (4; 4-2; 4-3) is arranged to generate an indication (I) that the pupil (14) is at a position suitable for acquiring the image (55) of the fundus (12); and
a controller (6) arranged to control the image acquisition apparatus (2) automatically to acquire the ultra-widefield image (55) of the fundus (12) in response to the patient alignment device (4; 4-2; 4-3) generating the indication (I).

4. The ultra-widefield ophthalmic imaging instrument (1; 100; 300) according to any preceding claim, wherein the patient alignment device (4-3) comprises a user interface (4d) by which the range of distances (R) used in the comparison is adjustable by a user of the ultra-widefield ophthalmic imaging instrument (1; 100; 300).

5. The ultra-widefield ophthalmic imaging instrument (1; 100; 300) according to any preceding claim, wherein the signals (S) for guiding the subject to vary the distance between the eye (10) and the image acquisition apparatus (2) to bring the pupil (14) towards the range of distances (R) from the exit pupil (E) comprise:
a projection of light of a first colour onto the eye (10) in case the subject is to move the eye (10) towards the image acquisition apparatus (2) to bring the pupil (14) towards the range of distances (R) from the exit pupil (E);
a projection of light of a second colour onto the eye (10) in case the subject is to move the eye (10) away from the image acquisition apparatus (2) to bring the pupil (14) towards the range of distances (R) from the exit pupil (E); and
a projection of light of a third colour onto the eye (10) in case the pupil (14) is within the range of distances (R) from the exit pupil (E) suitable for acquiring the image (55) of the fundus (12),
wherein the first colour, the second colour and the third colour are different from one another.

6. The ultra-widefield ophthalmic imaging instrument (1; 100; 300) according to Claim 5, wherein the patient alignment device (4; 4-2; 4-3) further comprises a fixation target light source (80) arranged to project each of the light of the first colour, the light of the second colour and the light of the third colour onto the eye (10) so as to fix a gaze direction of the eye (10) in a predetermined direction.

7. The ultra-widefield ophthalmic imaging instrument (1; 100; 300) according to Claim 6, wherein fixation target light source (80) is arranged to project each of the light of the first colour, the light of the second colour and the light of the third colour onto the eye (10) so as to have a projection on a fundus (12) of the eye (10) comprising at least one of cross hairs, a spot, a circle or one or more concentric circles.

8. The ultra-widefield ophthalmic imaging instrument (1; 100; 300) according to any preceding claim, wherein the ultra-widefield ophthalmic imaging instrument (100) comprises an ultra-widefield scanning laser ophthalmoscope.

9. A method of operating an ultra-widefield ophthalmic imaging instrument (100; 300) to acquire an ultra-widefield image (55) of a fundus (12) of an eye (10) of a subject, the method comprising:
monitoring (S10) a position of a pupil (14) of the eye (10) relative to an exit pupil (E) of the ultra-widefield ophthalmic imaging instrument (100; 300), by:
acquiring (S12) stereo images (46-1; 46-2) of the pupil (14); and
processing (S14) the stereo images (46-1; 46-2) of the pupil (14) to monitor the position of the pupil (14) relative to the exit pupil (E) of the ultra-widefield ophthalmic imaging instrument (100; 300);
comparing (S20) the position with a range of distances (R) from the exit pupil (E) suitable for acquiring the image (55) of the fundus (12) to determine whether the pupil (14) is within the range of distances (R) from the exit pupil (E), the range of distances (R) used in the comparison (S20) having been adjusted by:
determining (S2) an indication of a size of the pupil (14) based on at least one of the stereo images (46-1; 46-2) of the pupil (14);
adjusting (S4) the range of distances (R), based on the determined indication of the size of the pupil (14), such that the range of distances (R) increases as the size of the pupil (14) increases;
in case the pupil (14) is determined to be outside the range of distances (R), generating (S30), based on the monitored position, signals (S) for guiding the subject to vary a distance between the eye (10) and the ultra-widefield ophthalmic imaging instrument (100; 300) to bring the pupil (14) towards the range of distances (R) from the exit pupil (E);
in case the pupil (14) is determined to be within the range of distances (R), generating (S40) an indication that the pupil (14) is at a position suitable for acquiring the image (55) of the fundus (12); and
in response to the indication being generated, automatically controlling (S50) the ultra-widefield ophthalmic imaging instrument (100; 300) to acquire the ultra-widefield image (55) of the fundus (12).

10. The method according to Claim 9, wherein the signals (S) for guiding the subject to vary the distance between the eye (10) and the ultra-widefield ophthalmic imaging instrument (100; 300) to bring the pupil (14) towards the range of distances (R) from the exit pupil (E) comprise:
a projection of light of a first colour onto the eye (10) in case the subject is to move the eye (10) towards the ultra-widefield ophthalmic imaging instrument (100; 300) to bring the pupil (14) towards the range of distances (R) from the exit pupil (E);
a projection of light of a second colour onto the eye (10) in case the subject is to move the eye (10) away from the ultra-widefield ophthalmic imaging instrument (100; 300) to bring the pupil (14) towards the range of distances (R) from the exit pupil (E); and
a projection of light of a third colour onto the eye (10) in case the pupil (14) is within the range of distances (R) from the exit pupil (E) suitable for acquiring the image (55) of the fundus (12),
wherein the first colour, the second colour and the third colour are different from one another.

11. A method of operating an ultra-widefield ophthalmic imaging instrument (100; 300) to acquire an ultra-widefield image (55) of a fundus (12) of an eye (10) of a subject, the method comprising:
monitoring (S10) a position of a pupil (14) of the eye (10) relative to an exit pupil (E) of the ultra-widefield ophthalmic imaging instrument (100; 300), by using a distance sensor (4a) to measure a distance (L) to the eye (10) and generate a measurement signal (S_{M}) indicative of the measured distance (L), the position of the pupil (14) relative to the exit pupil (E) of the image acquisition apparatus (2) being monitored based on the measurement signal (S_{M});
comparing (S20) the position with a range of distances (R) from the exit pupil (E) suitable for acquiring the image (55) of the fundus (12) to determine whether the pupil (14) is within the range of distances (R) from the exit pupil (E), the range of distances (R) used in the comparison (S20) having been adjusted by:
using a camera (4c) to capture an image (M) of the pupil (14);
determining an indication of a size of the pupil (14) based on the captured image (M) of the pupil (14); and
using the determined indication of the size of the pupil (14) to adjust the range of distances (R) such that the range of distances (R) used in the comparison increases as the size of the pupil (14) increases;
in case the pupil (14) is determined to be outside the range of distances (R), generating (S30), based on the monitored position, signals (S) for guiding the subject to vary a distance between the eye (10) and the ultra-widefield ophthalmic imaging instrument (100; 300) to bring the pupil (14) towards the range of distances (R) from the exit pupil (E);
in case the pupil (14) is determined to be within the range of distances (R), generating (S40) an indication that the pupil (14) is at a position suitable for acquiring the image (55) of the fundus (12); and
in response to the indication being generated, automatically controlling (S50) the ultra-widefield ophthalmic imaging instrument (100; 300) to acquire the ultra-widefield image (55) of the fundus (12).

## Patentansprüche

1. Ophthalmisches Ultraweitfeld-Bildgebungsinstrument (1; 100; 300), umfassend:
eine Bilderfassungseinrichtung (2), die dazu ausgelegt ist, ein Ultraweitfeldbild (55) eines Fundus (12) eines Auges (10) einer Person über eine Austrittspupille (E) der Bilderfassungseinrichtung (2) zu erfassen;
eine Patientenausrichtungsvorrichtung (4; 4-2; 4-3), umfassend:
eine Stereobildgebungseinrichtung (40), die dazu ausgelegt ist, Stereobilder (46-1; 46-2) der Pupille (14) zu erfassen; und
einen Prozessor (50), der dazu ausgelegt ist, die Stereobilder (46-1; 46-2) zu verarbeiten, um eine Position einer Pupille (14) des Auges (10) relativ zu der Austrittspupille (E) der Bilderfassungseinrichtung (2) zu überwachen, und die Position mit einem Abstandsbereich (R) von der Austrittspupille (E), der zum Erfassen des Bildes (55) des Fundus (12) geeignet ist, zu vergleichen, um zu bestimmen, ob die Pupille (14) innerhalb des Abstandsbereichs (R) von der Austrittspupille (E) liegt, wobei der in dem Vergleich verwendete Abstandsbereich (R) einstellbar ist, wobei
der Prozessor (50) weiter dazu ausgelegt ist, eine Angabe einer Größe der Pupille (14), basierend auf mindestens einem der Stereobilder (46-1; 46-2) der Pupille (14), zu bestimmen und den in dem Vergleich der Position mit dem Abstandsbereich (R) verwendeten Abstandsbereich (R) einzustellen, basierend auf der bestimmten Angabe einer Größe der Pupille (14), derart, dass der in dem Vergleich verwendete Abstandsbereich (R) zunimmt, wenn die Größe der Pupille (14) zunimmt,
falls bestimmt wird, dass die Pupille (14) außerhalb des Abstandsbereichs (R) liegt, die Patientenausrichtungsvorrichtung (4; 4-2; 4-3) dazu ausgelegt ist, basierend auf der überwachten Position, Signale (S) zum Führen der Person zu erzeugen, um einen Abstand zwischen dem Auge (10) und der Bilderfassungseinrichtung (2) zu verändern, um die Pupille (14) in Richtung des Abstandsbereichs (R) von der Austrittspupille (E) zu bringen, und
falls bestimmt wird, dass die Pupille (14) innerhalb des Abstandsbereichs (R) liegt, die Patientenausrichtungsvorrichtung (4; 4-2; 4-3) dazu ausgelegt ist, eine Anzeige (I) zu erzeugen, dass die Pupille (14) sich an einer zum Erfassen des Bildes (55) des Fundus (12) geeigneten Position befindet; und
eine Steuereinheit (6), die dazu ausgelegt ist, die Bilderfassungseinrichtung (2) automatisch so zu steuern, dass sie das Ultraweitfeldbild (55) des Fundus (12) als Reaktion darauf erfasst, dass die Patientenausrichtungsvorrichtung (4; 4-2; 4-3) die Anzeige (I) erzeugt.

2. Ophthalmisches Ultraweitfeld-Bildgebungsinstrument (100; 300) nach Anspruch 1, wobei die Bilderfassungseinrichtung (2) dazu ausgelegt ist, das Ultraweitfeldbild (55) des Fundus (12) durch Scannen eines Lichtstrahls (L_{T}) über den Fundus (12) zu erfassen, und umfasst:
eine Lichtquelle (20; 20'), die dazu ausgelegt ist, den Lichtstrahl (L_{T}; L_{T}') auszusenden;
einen drehbaren Spiegel (30), der dazu ausgelegt ist, den Lichtstrahl (L_{T}; L_{T}') über den Fundus (12) zu scannen; und
einen ellipsoidischen Spiegel (32), über den der drehbare Spiegel (30) dazu ausgelegt ist, den Lichtstrahl (L_{T}; L_{T}') über den Fundus (12) zu scannen, wobei der ellipsoidische Spiegel (32) einen ersten Brennpunkt (32-1) und einen zweiten Brennpunkt (32-2) aufweist, wobei der drehbare Spiegel (30) dazu ausgelegt ist, den Lichtstrahl (L_{T}; L_{T}') über den ellipsoidischen Spiegel (32) über den ersten Brennpunkt (32-1) zu scannen, und die Pupille (14) des Auges (10) während der Verwendung des ophthalmischen Ultraweitfeld-Bildgebungsinstruments (100) an dem zweiten Brennpunkt (32-2) positioniert ist,
wobei die Steuereinheit (6) dazu ausgelegt ist, in einem Patientenausrichtungsmodus zu arbeiten, um zu bewirken, dass die Patientenausrichtungsvorrichtung (4) die Position der Pupille (14) relativ zu der Austrittspupille (E) der Bilderfassungseinrichtung (2) überwacht, und in einem Fundusbildgebungsmodus zu arbeiten, um die Bilderfassungseinrichtung (2) so zu steuern, dass sie das Ultraweitfeldbild (55) des Fundus (12) erfasst, wobei
die Stereobildgebungseinrichtung (40) dazu ausgelegt ist, die Stereobilder (46-1; 46-2) der Pupille (14) über den drehbaren Spiegel (30) und den ellipsoidischen Spiegel (32) zu erfassen,
die Steuereinheit (6) im Patientenausrichtungsmodus dazu ausgelegt ist:
den drehbaren Spiegel (30) so zu steuern, dass er in einer vorbestimmten Ausrichtung ortsfest ist; und
die Stereobildgebungseinrichtung (40) so zu steuern, dass sie die Stereobilder (46-1; 46-2) der Pupille (14) über den drehbaren Spiegel (30) und den ellipsoidischen Spiegel (32) erfasst, während der drehbare Spiegel (30) in der vorbestimmten Ausrichtung ortsfest ist, und
die Steuereinheit (6) im Fundusbildgebungsmodus dazu ausgelegt ist:
die Lichtquelle (20; 20') so zu steuern, dass sie den Lichtstrahl (L_{T}; L_{T}') aussendet; und
die Bilderfassungseinrichtung (2) so zu steuern, dass sie das Ultraweitfeldbild (55) des Fundus (12) erfasst, indem sie den drehbaren Spiegel (30) so steuert, dass dieser den Lichtstrahl (L_{T}; L_{T}') über den Fundus (12) über den ellipsoidischen Spiegel (32) scannt.

3. Ophthalmisches Ultraweitfeld-Bildgebungsinstrument (1; 100; 300), umfassend:
eine Bilderfassungseinrichtung (2), die dazu ausgelegt ist, ein Ultraweitfeldbild (55) eines Fundus (12) eines Auges (10) einer Person über eine Austrittspupille (E) der Bilderfassungseinrichtung (2) zu erfassen;
eine Patientenausrichtungsvorrichtung (4; 4-2; 4-3), umfassend:
einen Abstandssensor (4a), der dazu ausgelegt ist, einen Abstand (L) zu dem Auge (10) zu messen und ein Messsignal (S_{M}) zu erzeugen, das den gemessenen Abstand (L) angibt, wobei die Patientenausrichtungsvorrichtung (4-2) dazu ausgelegt ist, eine Position einer Pupille (14) des Auges (10) relativ zu der Austrittspupille (E) der Bilderfassungseinrichtung (2) basierend auf dem Messsignal (S_{M}) zu überwachen, und die Position mit einem Abstandsbereich (R) von der Austrittspupille (E), der zum Erfassen des Bildes (55) des Fundus (12) geeignet ist, zu vergleichen, um zu bestimmen, ob die Pupille (14) innerhalb des Abstandsbereichs (R) von der Austrittspupille (E) liegt, wobei der in dem Vergleich verwendete Abstandsbereich (R) einstellbar ist; und
eine Kamera (4c), die dazu ausgelegt ist, ein Bild (M) der Pupille (14) aufzunehmen, wobei die Patientenausrichtungsvorrichtung (4-2) weiter dazu ausgelegt ist, eine Angabe einer Größe der Pupille (14) basierend auf dem aufgenommenen Bild (M) der Pupille (14) zu bestimmen, und den in dem Vergleich der Position mit dem Abstandsbereich (R) verwendeten Abstandsbereich (R) einzustellen, basierend auf der bestimmten Angabe einer Größe der Pupille (14), derart, dass der in dem Vergleich verwendete Abstandsbereich (R) zunimmt, wenn die Größe der Pupille (14) zunimmt, wobei
falls bestimmt wird, dass die Pupille (14) außerhalb des Abstandsbereichs (R) liegt, die Patientenausrichtungsvorrichtung (4; 4-2; 4-3) dazu ausgelegt ist, basierend auf der überwachten Position, Signale (S) zum Führen der Person zu erzeugen, um einen Abstand zwischen dem Auge (10) und der Bilderfassungseinrichtung (2) zu verändern, um die Pupille (14) in Richtung des Abstandsbereichs (R) von der Austrittspupille (E) zu bringen, und
falls bestimmt wird, dass die Pupille (14) innerhalb des Abstandsbereichs (R) liegt, die Patientenausrichtungsvorrichtung (4; 4-2; 4-3) dazu ausgelegt ist, eine Anzeige (I) zu erzeugen, dass die Pupille (14) sich an einer zum Erfassen des Bildes (55) des Fundus (12) geeigneten Position befindet; und
eine Steuereinheit (6), die dazu ausgelegt ist, die Bilderfassungseinrichtung (2) automatisch so zu steuern, dass sie das Ultraweitfeldbild (55) des Fundus (12) als Reaktion darauf erfasst, dass die Patientenausrichtungsvorrichtung (4; 4-2; 4-3) die Anzeige (I) erzeugt.

4. Ophthalmisches Ultraweitfeld-Bildgebungsinstrument (1; 100; 300) nach einem vorstehenden Anspruch, wobei die Patientenausrichtungsvorrichtung (4-3) eine Benutzerschnittstelle (4d) umfasst, durch die der in dem Vergleich verwendete Abstandsbereich (R) durch einen Benutzer des ophthalmischen Ultraweitfeld-Bildgebungsinstruments (1; 100; 300) einstellbar ist.

5. Ophthalmisches Ultraweitfeld-Bildgebungsinstrument (1; 100; 300) nach einem vorstehenden Anspruch, wobei die Signale (S) zum Führen der Person, um den Abstand zwischen dem Auge (10) und der Bilderfassungseinrichtung (2) zu verändern, um die Pupille (14) in Richtung des Abstandsbereichs (R) von der Austrittspupille (E) zu bringen, umfassen:
eine Projektion von Licht einer ersten Farbe auf das Auge (10), falls die Person das Auge (10) in Richtung der Bilderfassungseinrichtung (2) bewegen soll, um die Pupille (14) in Richtung des Abstandsbereichs (R) von der Austrittspupille (E) zu bringen;
eine Projektion von Licht einer zweiten Farbe auf das Auge (10), falls die Person das Auge (10) von der Bilderfassungseinrichtung (2) wegbewegen soll, um die Pupille (14) in Richtung des Abstandsbereichs (R) von der Austrittspupille (E) zu bringen; und
eine Projektion von Licht einer dritten Farbe auf das Auge (10), falls die Pupille (14) innerhalb des Abstandsbereichs (R) von der Austrittspupille (E) liegt, der zum Erfassen des Bildes (55) des Fundus (12) geeignet ist,
wobei die erste Farbe, die zweite Farbe und die dritte Farbe voneinander verschieden sind.

6. Ophthalmisches Ultraweitfeld-Bildgebungsinstrument (1; 100; 300) nach Anspruch 5, wobei die Patientenausrichtungsvorrichtung (4; 4-2; 4-3) weiter eine Fixationsziel-Lichtquelle (80) umfasst, die dazu ausgelegt ist, jedes von dem Licht der ersten Farbe, dem Licht der zweiten Farbe und dem Licht der dritten Farbe auf das Auge (10) zu projizieren, um eine Blickrichtung des Auges (10) in einer vorbestimmten Richtung zu fixieren.

7. Ophthalmisches Ultraweitfeld-Bildgebungsinstrument (1; 100; 300) nach Anspruch 6, wobei Fixationsziel-Lichtquelle (80) dazu ausgelegt ist, jedes von dem Licht der ersten Farbe, dem Licht der zweiten Farbe und dem Licht der dritten Farbe auf das Auge (10) zu projizieren, sodass sie eine Projektion auf einem Fundus (12) des Auges (10) aufweist, die mindestens eines von Fadenkreuzen, einem Punkt, einem Kreis oder einem oder mehreren konzentrischen Kreisen umfasst.

8. Ophthalmisches Ultraweitfeld-Bildgebungsinstrument (1; 100; 300) nach einem vorstehenden Anspruch, wobei das ophthalmische Ultraweitfeld-Bildgebungsinstrument (100) ein Ultraweitfeld-Rasterlaser-Ophthalmoskop umfasst.

9. Verfahren zum Betreiben eines ophthalmischen Ultraweitfeld-Bildgebungsinstruments (100; 300) zum Erfassen eines Ultraweitfeldbildes (55) eines Fundus (12) eines Auges (10) einer Person, wobei das Verfahren umfasst:
Überwachen (S10) einer Position einer Pupille (14) des Auges (10) relativ zu einer Austrittspupille (E) des ophthalmischen Ultraweitfeld-Bildgebungsinstruments (100; 300) durch:
Erfassen (S12) von Stereobildern (46-1; 46-2) der Pupille (14); und
Verarbeiten (S14) der Stereobilder (46-1; 46-2) der Pupille (14), um die Position der Pupille (14) relativ zu der Austrittspupille (E) des ophthalmischen Ultraweitfeld-Bildgebungsinstruments (100; 300) zu überwachen;
Vergleichen (S20) der Position mit einem Abstandsbereich (R) von der Austrittspupille (E), der zum Erfassen des Bildes (55) des Fundus (12) geeignet ist, um zu bestimmen, ob die Pupille (14) innerhalb des Abstandsbereichs (R) von der Austrittspupille (E) liegt, wobei der in dem Vergleich (S20) verwendete Abstandsbereich (R) eingestellt worden ist durch:
Bestimmen (S2) einer Angabe einer Größe der Pupille (14) basierend auf mindestens einem der Stereobilder (46-1; 46-2) der Pupille (14);
Einstellen (S4) des Abstandsbereichs (R), basierend auf der bestimmten Angabe einer Größe der Pupille (14), derart, dass der Abstandsbereich (R) zunimmt, wenn die Größe der Pupille (14) zunimmt;
falls bestimmt wird, dass die Pupille (14) außerhalb des Abstandsbereichs (R) liegt, Erzeugen (S30) von Signalen (S), basierend auf der überwachten Position, zum Führen der Person, um einen Abstand zwischen dem Auge (10) und dem ophthalmischen Ultraweitfeld-Bildgebungsinstrument (100; 300) zu verändern, um die Pupille (14) in Richtung des Abstandsbereichs (R) von der Austrittspupille (E) zu bringen;
falls bestimmt wird, dass die Pupille (14) innerhalb des Abstandsbereichs (R) liegt, Erzeugen (S40) einer Anzeige, dass die Pupille (14) sich an einer zum Erfassen des Bildes (55) des Fundus (12) geeigneten Position befindet; und
automatisches Steuern (S50) des ophthalmischen Ultraweitfeld-Bildgebungsinstruments (100; 300), um das Ultraweitfeldbild (55) des Fundus (12) zu erfassen, als Reaktion darauf, dass die Anzeige erzeugt wird.

10. Verfahren nach Anspruch 9, wobei die Signale (S) zum Führen der Person, um den Abstand zwischen dem Auge (10) und dem ophthalmischen Ultraweitfeld-Bildgebungsinstrument (100; 300) zu verändern, um die Pupille (14) in Richtung des Abstandsbereichs (R) von der Austrittspupille (E) zu bringen, umfassen:
eine Projektion von Licht einer ersten Farbe auf das Auge (10), falls die Person das Auge (10) in Richtung des ophthalmischen Ultraweitfeld-Bildgebungsinstruments (100; 300) bewegen soll, um die Pupille (14) in Richtung des Abstandsbereichs (R) von der Austrittspupille (E) zu bringen;
eine Projektion von Licht einer zweiten Farbe auf das Auge (10), falls die Person das Auge (10) von dem ophthalmischen Ultraweitfeld-Bildgebungsinstrument (100; 300) wegbewegen soll, um die Pupille (14) in Richtung des Abstandsbereichs (R) von der Austrittspupille (E) zu bringen; und
eine Projektion von Licht einer dritten Farbe auf das Auge (10), falls die Pupille (14) innerhalb des Abstandsbereichs (R) von der Austrittspupille (E) liegt, der zum Erfassen des Bildes (55) des Fundus (12) geeignet ist,
wobei die erste Farbe, die zweite Farbe und die dritte Farbe voneinander verschieden sind.

11. Verfahren zum Betreiben eines ophthalmischen Ultraweitfeld-Bildgebungsinstruments (100; 300) zum Erfassen eines Ultraweitfeldbildes (55) eines Fundus (12) eines Auges (10) einer Person, wobei das Verfahren umfasst:
Überwachen (S10) einer Position einer Pupille (14) des Auges (10) relativ zu einer Austrittspupille (E) des ophthalmischen Ultraweitfeld-Bildgebungsinstruments (100; 300), unter Verwendung eines Abstandssensors (4a), um einen Abstand (L) zu dem Auge (10) zu messen und ein Messsignal (S_{M}) zu erzeugen, das den gemessenen Abstand (L) angibt, wobei die Position der Pupille (14) relativ zu der Austrittspupille (E) der Bilderfassungseinrichtung (2) basierend auf dem Messsignal (S_{M}) überwacht wird;
Vergleichen (S20) der Position mit einem Abstandsbereich (R) von der Austrittspupille (E), der zum Erfassen des Bildes (55) des Fundus (12) geeignet ist, um zu bestimmen, ob die Pupille (14) innerhalb des Abstandsbereichs (R) von der Austrittspupille (E) liegt, wobei der in dem Vergleich (S20) verwendete Abstandsbereich (R) eingestellt worden ist durch:
Verwenden einer Kamera (4c), um ein Bild (M) der Pupille (14) aufzunehmen;
Bestimmen einer Angabe einer Größe der Pupille (14) basierend auf dem aufgenommenen Bild (M) der Pupille (14); und
Verwenden der bestimmten Angabe einer Größe der Pupille (14), um den Abstandsbereich (R) so einzustellen, dass der in dem Vergleich verwendete Abstandsbereich (R) zunimmt, wenn die Größe der Pupille (14) zunimmt;
falls bestimmt wird, dass die Pupille (14) außerhalb des Abstandsbereichs (R) liegt, Erzeugen (S30) von Signalen (S), basierend auf der überwachten Position, zum Führen der Person, um einen Abstand zwischen dem Auge (10) und dem ophthalmischen Ultraweitfeld-Bildgebungsinstrument (100; 300) zu verändern, um die Pupille (14) in Richtung des Abstandsbereichs (R) von der Austrittspupille (E) zu bringen;
falls bestimmt wird, dass die Pupille (14) innerhalb des Abstandsbereichs (R) liegt, Erzeugen (S40) einer Anzeige, dass die Pupille (14) sich an einer zum Erfassen des Bildes (55) des Fundus (12) geeigneten Position befindet; und
automatisches Steuern (S50) des ophthalmischen Ultraweitfeld-Bildgebungsinstruments (100; 300), um das Ultraweitfeldbild (55) des Fundus (12) zu erfassen, als Reaktion darauf, dass die Anzeige erzeugt wird.

## Revendications

1. Instrument d'imagerie ophtalmique à ultra-grand champ (1 ; 100 ; 300) comprenant :
un appareil d'acquisition d'image (2) agencé pour acquérir une image ultra-grand champ (55) d'un fond d'œil (12) d'un œil (10) d'un sujet via une pupille de sortie (E) de l'appareil d'acquisition d'image (2) ;
un dispositif d'alignement de patient (4 ; 4-2 ; 4-3) comprenant :
un appareil d'imagerie stéréoscopique (40) agencé pour acquérir des images stéréoscopiques (46-1 ; 46-2) de la pupille (14) ; et
un processeur (50) agencé pour traiter les images stéréoscopiques (46-1 ; 46-2) pour surveiller une position d'une pupille (14) de l'œil (10) par rapport à la pupille de sortie (E) de l'appareil d'acquisition d'image (2), et
comparer la position avec une plage de distances (R) à partir de la pupille de sortie (E) adaptée pour acquérir l'image (55) du fond d'œil (12) afin de déterminer si la pupille (14) est à l'intérieur de la plage de distances (R) à partir de la pupille de sortie (E), la plage de distances (R) utilisée dans la comparaison étant ajustable, dans lequel
le processeur (50) est en outre agencé pour déterminer une indication d'une taille de la pupille (14) sur la base d'au moins une des images stéréoscopiques (46-1 ; 46-2) de la pupille (14), et pour ajuster la plage de distances (R) utilisée dans la comparaison de la position avec la plage de distances (R), sur la base de l'indication déterminée de la taille de la pupille (14), de telle sorte que la plage de distances (R) utilisée dans la comparaison augmente à mesure que la taille de la pupille (14) augmente,
dans le cas où la pupille (14) est déterminée comme étant à l'extérieur de la plage de distances (R), le dispositif d'alignement de patient (4 ; 4-2 ; 4-3) est agencé pour générer, sur la base de la position surveillée, des signaux (S) pour guider le sujet afin de faire varier une distance entre l'œil (10) et l'appareil d'acquisition d'image (2) pour amener la pupille (14) vers la plage de distances (R) à partir de la pupille de sortie (E), et
dans le cas où la pupille (14) est déterminée comme étant à l'intérieur de la plage de distances (R), le dispositif d'alignement de patient (4 ; 4-2 ; 4-3) est agencé pour générer une indication (I) selon laquelle la pupille (14) est à une position adaptée pour acquérir l'image (55) du fond d'œil (12) ; et
une unité de commande (6) agencée pour commander automatiquement l'appareil d'acquisition d'image (2) pour acquérir l'image ultra-grand champ (55) du fond d'œil (12) en réponse au dispositif d'alignement de patient (4 ; 4-2 ; 4-3) générant l'indication (I).

2. Instrument d'imagerie ophtalmique à ultra-grand champ (100 ; 300) selon la revendication 1, dans lequel l'appareil d'acquisition d'image (2) est agencé pour acquérir l'image ultra-grand champ (55) du fond d'œil (12) par balayage d'un faisceau lumineux (L_{T}) à travers le fond d'œil (12), et comprend :
une source lumineuse (20 ; 20') agencée pour émettre le faisceau lumineux (L_{T} ; L_{T'}) ;
un miroir rotatif (30) agencé pour balayer le faisceau lumineux (L_{T} ; L_{T'}) à travers le fond d'œil (12) ; et
un miroir ellipsoïdal (32) via lequel le miroir rotatif (30) est agencé pour balayer le faisceau lumineux (L_{T} ; L_{T'}) à travers le fond d'œil (12), le miroir ellipsoïdal (32) présentant un premier foyer (32-1) et un deuxième foyer (32-2),
dans lequel le miroir rotatif (30) est agencé pour balayer le faisceau lumineux (L_{T} ; L_{T'}) à travers le miroir ellipsoïdal (32) via le premier foyer (32-1), et la pupille (14) de l'œil (10) est positionnée au niveau du deuxième foyer (32-2) pendant l'utilisation de l'instrument d'imagerie ophtalmique à ultra-grand champ (100),
dans lequel l'unité de commande (6) est agencée pour fonctionner dans un mode d'alignement de patient pour amener le dispositif d'alignement de patient (4) à surveiller la position de la pupille (14) par rapport à la pupille de sortie (E) de l'appareil d'acquisition d'image (2), et pour fonctionner dans un mode d'imagerie de fond d'œil pour commander l'appareil d'acquisition d'image (2) pour acquérir l'image ultra-grand champ (55) du fond d'œil (12), dans lequel
l'appareil d'imagerie stéréoscopique (40) est agencé pour acquérir les images stéréoscopiques (46-1 ; 46-2) de la pupille (14) via le miroir rotatif (30) et le miroir ellipsoïdal (32), dans le mode d'alignement de patient, l'unité de commande (6) est agencée pour :
commander le miroir rotatif (30) pour être immobile dans une orientation prédéterminée ; et
commander l'appareil d'imagerie stéréoscopique (40) pour acquérir les images stéréoscopiques (46-1 ; 46-2) de la pupille (14) via le miroir rotatif (30) et le miroir ellipsoïdal (32) tandis que le miroir rotatif (30) est immobile dans l'orientation prédéterminée, et
dans le mode d'imagerie de fond d'œil, l'unité de commande (6) est agencée pour :
commander la source lumineuse (20 ; 20') pour émettre le faisceau lumineux (L_{T} ; L_{T'}) ; et
commander l'appareil d'acquisition d'image (2) pour acquérir l'image ultra-grand champ (55) du fond d'œil (12) par commande du miroir rotatif (30) pour balayer le faisceau lumineux (L_{T} ; L_{T'}) à travers le fond d'œil (12) via le miroir ellipsoïdal (32).

3. Instrument d'imagerie ophtalmique à ultra-grand champ (1 ; 100 ; 300) comprenant :
un appareil d'acquisition d'image (2) agencé pour acquérir une image ultra-grand champ (55) d'un fond d'œil (12) d'un œil (10) d'un sujet via une pupille de sortie (E) de l'appareil d'acquisition d'image (2) ;
un dispositif d'alignement de patient (4 ; 4-2 ; 4-3) comprenant :
un capteur de distance (4a) agencé pour mesurer une distance (L) jusqu'à l'œil (10) et générer un signal de mesure (S_{M}) indicatif de la distance (L) mesurée, le dispositif d'alignement de patient (4-2) étant agencé pour surveiller une position d'une pupille (14) de l'œil (10) par rapport à la pupille de sortie (E) de l'appareil d'acquisition d'image (2) sur la base du signal de mesure (S_{M}), et
comparer la position avec une plage de distances (R) à partir de la pupille de sortie (E) adaptée pour acquérir l'image (55) du fond d'œil (12) afin de déterminer si la pupille (14) est à l'intérieur de la plage de distances (R) à partir de la pupille de sortie (E), la plage de distances (R) utilisée dans la comparaison étant ajustable ; et
une caméra (4c) agencée pour capturer une image (M) de la pupille (14), le dispositif d'alignement de patient (4-2) étant en outre agencé pour déterminer une indication d'une taille de la pupille (14) sur la base de l'image capturée (M) de la pupille (14), et
ajuster la plage de distances (R) utilisée dans la comparaison de la position avec la plage de distances (R), sur la base de l'indication déterminée de la taille de la pupille (14), de telle sorte que la plage de distances (R) utilisée dans la comparaison augmente à mesure que la taille de la pupille (14) augmente, dans lequel
dans le cas où la pupille (14) est déterminée comme étant à l'extérieur de la plage de distances (R), le dispositif d'alignement de patient (4 ; 4-2 ; 4-3) est agencé pour générer, sur la base de la position surveillée, des signaux (S) pour guider le sujet afin de faire varier une distance entre l'œil (10) et l'appareil d'acquisition d'image (2) pour amener la pupille (14) vers la plage de distances (R) à partir de la pupille de sortie (E), et
dans le cas où la pupille (14) est déterminée comme étant à l'intérieur de la plage de distances (R), le dispositif d'alignement de patient (4 ; 4-2 ; 4-3) est agencé pour générer une indication (I) selon laquelle la pupille (14) est à une position adaptée pour acquérir l'image (55) du fond d'œil (12) ; et
une unité de commande (6) agencée pour commander automatiquement l'appareil d'acquisition d'image (2) pour acquérir l'image ultra-grand champ (55) du fond d'œil (12) en réponse au dispositif d'alignement de patient (4 ; 4-2 ; 4-3) générant l'indication (I).

4. Instrument d'imagerie ophtalmique à ultra-grand champ (1 ; 100 ; 300) selon l'une quelconque des revendications précédentes, dans lequel le dispositif d'alignement de patient (4-3) comprend une interface utilisateur (4d) au moyen de laquelle la plage de distances (R) utilisée dans la comparaison est ajustable par un utilisateur de l'instrument d'imagerie ophtalmique à ultra-grand champ (1 ; 100 ; 300).

5. Instrument d'imagerie ophtalmique à ultra-grand champ (1 ; 100 ; 300) selon l'une quelconque des revendications précédentes, dans lequel les signaux (S) pour guider le sujet afin de faire varier la distance entre l'œil (10) et l'appareil d'acquisition d'image (2) pour amener la pupille (14) vers la plage de distances (R) à partir de la pupille de sortie (E) comprennent :
une projection de lumière d'une première couleur sur l'œil (10) dans le cas où le sujet doit déplacer l'œil (10) vers l'appareil d'acquisition d'image (2) pour amener la pupille (14) vers la plage de distances (R) à partir de la pupille de sortie (E) ;
une projection de lumière d'une deuxième couleur sur l'œil (10) dans le cas où le sujet doit déplacer l'œil (10) à l'écart de l'appareil d'acquisition d'image (2) pour amener la pupille (14) vers la plage de distances (R) à partir de la pupille de sortie (E) ; et
une projection de lumière d'une troisième couleur sur l'œil (10) dans le cas où la pupille (14) est à l'intérieur de la plage de distances (R) à partir de la pupille de sortie (E) adaptée pour acquérir l'image (55) du fond d'œil (12),
dans lequel la première couleur, la deuxième couleur et la troisième couleur sont différentes les unes des autres.

6. Instrument d'imagerie ophtalmique à ultra-grand champ (1 ; 100 ; 300) selon la revendication 5, dans lequel le dispositif d'alignement de patient (4 ; 4-2 ; 4-3) comprend en outre une source lumineuse de cible de fixation (80) agencée pour projeter chaque lumière parmi la lumière de la première couleur, la lumière de la deuxième couleur et la lumière de la troisième couleur sur l'œil (10) de manière à fixer une direction du regard de l'œil (10) dans une direction prédéterminée.

7. Instrument d'imagerie ophtalmique à ultra-grand champ (1 ; 100 ; 300) selon la revendication 6, dans lequel la source lumineuse de cible de fixation (80) est agencée pour projeter chaque lumière parmi la lumière de la première couleur, la lumière de la deuxième couleur et la lumière de la troisième couleur sur l'œil (10) de manière à avoir une projection sur un fond d'œil (12) de l'œil (10) comprenant au moins un élément parmi un réticule, un point, un cercle ou un ou plusieurs cercles concentriques.

8. Instrument d'imagerie ophtalmique à ultra-grand champ (1 ; 100 ; 300) selon l'une quelconque des revendications précédentes, dans lequel l'instrument d'imagerie ophtalmique à ultra-grand champ (100) comprend un ophtalmoscope laser à balayage à ultra-grand champ.

9. Procédé de fonctionnement d'un instrument d'imagerie ophtalmique à ultra-grand champ (100 ; 300) pour acquérir une image ultra-grand champ (55) d'un fond d'œil (12) d'un œil (10) d'un sujet, le procédé comprenant :
surveiller (S10) une position d'une pupille (14) de l'œil (10) par rapport à une pupille de sortie (E) de l'instrument d'imagerie ophtalmique à ultra-grand champ (100 ; 300), par :
acquérir (S12) des images stéréoscopiques (46-1 ; 46-2) de la pupille (14) ; et
traiter (S14) les images stéréoscopiques (46-1 ; 46-2) de la pupille (14) pour surveiller la position de la pupille (14) par rapport à la pupille de sortie (E) de l'instrument d'imagerie ophtalmique à ultra-grand champ (100 ; 300) ;
comparer (S20) la position avec une plage de distances (R) à partir de la pupille de sortie (E) adaptée pour acquérir l'image (55) du fond d'œil (12) afin de déterminer si la pupille (14) est à l'intérieur de la plage de distances (R) à partir de la pupille de sortie (E), la plage de distances (R) utilisée dans la comparaison (S20) ayant été ajustée par :
déterminer (S2) une indication d'une taille de la pupille (14) sur la base d'au moins une des images stéréoscopiques (46-1 ; 46-2) de la pupille (14) ;
ajuster (S4) la plage de distances (R), sur la base de l'indication déterminée de la taille de la pupille (14), de telle sorte que la plage de distances (R) augmente à mesure que la taille de la pupille (14) augmente ;
dans le cas où la pupille (14) est déterminée comme étant à l'extérieur de la plage de distances (R), générer (S30), sur la base de la position surveillée, des signaux (S) pour guider le sujet afin de faire varier une distance entre l'œil (10) et l'instrument d'imagerie ophtalmique à ultra-grand champ (100 ; 300) pour amener la pupille (14) vers la plage de distances (R) à partir de la pupille de sortie (E) ;
dans le cas où la pupille (14) est déterminée comme étant à l'intérieur de la plage de distances (R), générer (S40) une indication selon laquelle la pupille (14) est à une position adaptée pour acquérir l'image (55) du fond d'œil (12) ; et
en réponse à la génération de l'indication, commander automatiquement (S50) l'instrument d'imagerie ophtalmique à ultra-grand champ (100 ; 300) pour acquérir l'image ultra-grand champ (55) du fond d'œil (12).

10. Procédé selon la revendication 9, dans lequel les signaux (S) pour guider le sujet afin de faire varier la distance entre l'œil (10) et l'instrument d'imagerie ophtalmique à ultra-grand champ (100 ; 300) pour amener la pupille (14) vers la plage de distances (R) à partir de la pupille de sortie (E) comprennent :
une projection de lumière d'une première couleur sur l'œil (10) dans le cas où le sujet doit déplacer l'œil (10) vers l'instrument d'imagerie ophtalmique à ultra-grand champ (100 ; 300) pour amener la pupille (14) vers la plage de distances (R) à partir de la pupille de sortie (E) ;
une projection de lumière d'une deuxième couleur sur l'œil (10) dans le cas où le sujet doit déplacer l'œil (10) à l'écart de l'instrument d'imagerie ophtalmique à ultra-grand champ (100 ; 300) pour amener la pupille (14) vers la plage de distances (R) à partir de la pupille de sortie (E) ; et
une projection de lumière d'une troisième couleur sur l'œil (10) dans le cas où la pupille (14) est à l'intérieur de la plage de distances (R) à partir de la pupille de sortie (E) adaptée pour acquérir l'image (55) du fond d'œil (12),
dans lequel la première couleur, la deuxième couleur et la troisième couleur sont différentes les unes des autres.

11. Procédé de fonctionnement d'un instrument d'imagerie ophtalmique à ultra-grand champ (100 ; 300) pour acquérir une image ultra-grand champ (55) d'un fond d'œil (12) d'un œil (10) d'un sujet, le procédé comprenant :
surveiller (S10) une position d'une pupille (14) de l'œil (10) par rapport à une pupille de sortie (E) de l'instrument d'imagerie ophtalmique à ultra-grand champ (100 ; 300), en utilisant un capteur de distance (4a) pour mesurer une distance (L) jusqu'à l'œil (10) et générer un signal de mesure (S_{M}) indicatif de la distance (L) mesurée, la position de la pupille (14) par rapport à la pupille de sortie (E) de l'appareil d'acquisition d'image (2) étant surveillée sur la base du signal de mesure (S_{M}) ;
comparer (S20) la position avec une plage de distances (R) à partir de la pupille de sortie (E) adaptée pour acquérir l'image (55) du fond d'œil (12) afin de déterminer si la pupille (14) est à l'intérieur de la plage de distances (R) à partir de la pupille de sortie (E), la plage de distances (R) utilisée dans la comparaison (S20) ayant été ajustée par :
utiliser une caméra (4c) pour capturer une image (M) de la pupille (14) ;
déterminer une indication d'une taille de la pupille (14) sur la base de l'image capturée (M) de la pupille (14) ; et
utiliser l'indication déterminée de la taille de la pupille (14) pour ajuster la plage de distances (R) de telle sorte que la plage de distances (R) utilisée dans la comparaison augmente à mesure que la taille de la pupille (14) augmente ;
dans le cas où la pupille (14) est déterminée comme étant à l'extérieur de la plage de distances (R),
générer (S30), sur la base de la position surveillée, des signaux (S) pour guider le sujet afin de faire varier une distance entre l'œil (10) et l'instrument d'imagerie ophtalmique à ultra-grand champ (100 ; 300) pour amener la pupille (14) vers la plage de distances (R) à partir de la pupille de sortie (E) ;
dans le cas où la pupille (14) est déterminée comme étant à l'intérieur de la plage de distances (R), générer (S40) une indication selon laquelle la pupille (14) est à une position adaptée pour acquérir l'image (55) du fond d'œil (12) ; et
en réponse à la génération de l'indication, commander automatiquement (S50) l'instrument d'imagerie ophtalmique à ultra-grand champ (100 ; 300) pour acquérir l'image ultra-grand champ (55) du fond d'œil (12).
